# EUROPEAN PATENT APPLICATION

(11) **EP 4 454 677 A2**
(43) Date of publication of application: **30.10.2024**
(21) Application number: 24200578.3
(22) Date of filing: 27.02.2008
(51) Int. Cl.: A61M 1/36

(54) **SENSOR APPARATUS SYSTEMS, DEVICES AND METHODS**

(30) Priority: 27.02.2007 US 904024 P; 02.04.2007 US 921314 P; 12.10.2007 US 871821
(62) Divisional of application: 20184472.7
(71) Applicant: DEKA Products Limited Partnership, Manchester, NH 03101-1108 (US)
(72) Inventor: Kamen, Dean, Bedford, NH, 03110 (US); Perry, N. Christopher, Manchester, NH, 03104 (US); Demers, Jason A., Manchester, NH, 03104 (US); Tracey, Brian, Litchfield, NH, 03052 (US); Chawan, Arun D., San Francisco, California, 94110 (US); Grant, Kevin L., Litchfield, NH, 03052 (US)
(74) Representative: Hoffmann Eitle

(57) **Abstract**

There is disclosed herein a sensor manifold (4100) for sensing liquid in a sensor apparatus, the sensor manifold comprising: a base (4301) with a plurality of fluid channels (4220, 4221, 4223, 4224, 4225) in fluid communication with a plurality of tube connectors (4101, 4113); a top plate (4302) with a plurality of ports, the top plate being sealingly engaged with the base to define the plurality of fluid channels, each of the plurality of ports configured to receive a sensing probe (4501) that extends into one of the plurality of fluid paths; and a printed circuit board (PCB) (4502) electrically connected to each of the sensing probes; wherein the top plate is positioned between the printed circuit board and the base, the PCB, top plate, and base are held together as a unit, and there are at least two sensing probes positioned in each fluid path for measuring conductivity of liquid in each fluid path.

## Description

### Cross-Reference to Related Applications

This application is a continuation-in-part of Patent Application Serial No.: 11/871,821, filed October 12, 2007 and entitled **Sensor Apparatus Systems, Devices and Methods,** which application claims priority from the following United States Provisional Patent Applications, all of which are hereby incorporated herein by reference in their entireties:
U.S. Provisional Patent Application No. 60/904,024 entitled Hemodialysis System and Methods filed on February 27, 2007; and
U.S. Provisional Patent Application No. 60/921,314 entitled Sensor Apparatus filed on April 2, 2007.

This application is also related to the following United States Patent Applications, and are hereby incorporated herein by reference in their entireties:
U.S. Patent Application Serial No. 11/871,712, filed October 12, 2007 entitled **Pumping Cassette** (Attorney Docket No. DEKA-020XX); U.S. Patent Application Serial No. 11/871,787, filed October 12, 2007 and entitled **Pumping Cassette** (Attorney Docket No. DEKA-021XX); U.S. Patent Application Serial No. 11/871,793, filed October 12, 2007 and entitled **Pumping Cassette** (Attorney Docket No. DEKA-022XX); U.S. Patent Application Serial No. 11/871,803, filed October 12, 2007 and entitled **Cassette System Integrated Apparatus** (Attorney Docket No. DEKA-023XX); and U.S. Patent Application Serial No. 11/871,828, filed October 12, 2007 and entitled **Peritoneal Dialysis Sensor Apparatus, Systems, Devices and Methods** (Attorney Docket: F30 (previously DEKA-025XX)).

Further, this application is related to the following United States Patent Applications, which are being filed on even date herewith and are hereby incorporated herein by reference in their entireties:
U.S. Patent Application entitled **Cassette System Integrated Apparatus** (Attorney Docket No. F62); and U.S. Patent Application entitled **Hemodialysis System and Methods** (Attorney Docket No. D0570/70019US00).

### Technical Field

The present invention relates to sensor systems, devices, and methods, and more particularly to systems, devices, and methods for sensors, sensor apparatus, and sensor apparatus systems.

### Background Art

In many applications, the temperature of a media, whether a solid, liquid or gas, is determined. One method is introducing a temperature sensor apparatus or probe to the medium being measured. For accuracy, close proximity of the sensor to the subject media is desired. However, this method may lead to contamination of the sensor apparatus and/or the fluid. Additional problems with harsh media or problems with the accuracy of the device used exist.

The concentration of a known compound in a media, whether fluid or otherwise, can be determined through measuring the conductivity of the fluid. Determining the conductivity of a material can also provide useful information such as the composition or presence of a particular compound in a material or irregularities in the conductive material between conductivity sensing probes. The presence, absence or variation of conductivity can also be a useful determinant of anomalies in a system.

There is a need for an apparatus that can both sense the temperature and the conductivity of a fluid or other media. There is a desire for a combination temperature and conductivity sensor that avoid contamination with the subject media and is compact. Also, there is a desire for an accurate temperature sensing device.

Additionally, there is a need for an accurate measurement apparatus to measure the temperature, conductivity, and/or other condition of a subject media while avoiding contamination between with the measurement apparatus and the subject media. There is also a need for an accurate measurement apparatus that can measure the temperature, conductivity, and/or other condition of a subject media where such subject media is contained in and/or flowing through a disposable component such that part or all of the sensor apparatus can be reused and need not be disposed of along with the disposable component.

### Summary of the Invention

In accordance with one aspect of the invention there is provided a sensor apparatus system for determining one or more properties of a subject fluid in a cassette, the system comprising a probe housing; a thermal sensor in said probe housing having a sensing end and a connector end; a probe tip thermally coupled to said sensing end of the thermal sensor and attached to said probe housing, the probe tip adapted for thermal coupling with an inner surface of a well installed in a cassette; and at least two leads connected to said connector end of said thermal sensor, whereby thermal energy is transferred from said well to said thermal sensor and whereby temperature information is conveyed through said leads. In various alternative embodiments, the sensing probe may further include a third lead attached to one of the probe housing, the thermal sensor, and the probe tip for permitting conductivity sensing. Alternatively, the sensing probe may further include a conductivity sensor attached to one of the probe housing, the thermal sensor, and the probe tip for permitting conductivity sensing; and a third lead attached to the conductivity sensor for transmitting conductivity information. A urethane resin may be included between said probe tip and said probe housing. The probe tip may include a flange for mating with the housing.

In various alternative embodiments of the sensor apparatus system described above, thermal epoxy may be included between said thermal sensor and said probe tip. The probe tip may be copper, steel, or a metal including at least one of silver, copper, steel, and stainless steel. In various embodiments, the housing may be plastic or metal. The housing may include a flange disposed about said probe housing, and a spring may be used in conjunction with the flange. The housing may include an integrated flexible member.

Some embodiments of this aspect of the present invention include a well of a predetermined size and shape. The well mates with the probe and the probe tip is thermal coupled to said well.

In accordance with one aspect of the present invention the well includes a hollow housing of a thermally conductive material. The housing has an outer surface and an inner surface. The inner surface is a predetermined shape so as to form a mating relationship with a sensing probe. The mating thermally couples the inner surface with a sensing probe.

Some embodiments of this aspect of the present invention include a predetermined volume of thermal grease on the inner surface of the well.

In accordance with one aspect of the present invention, method for determining temperature and conductivity of a subject media in a cassette is described. The method includes the following steps: installing at least one well in a cassette; thermally coupling a well and a sensing probe such that temperature and conductivity can be determined; transferring thermal and conductivity signals through at least 3 leads from the sensing probe; and determining temperature and conductivity using the signals.

In accordance with another aspect of the present invention, a method for detecting air in a fluid line contained in a cassette is described. The method includes the following steps: installing at least one well in a cassette; thermally coupling at least two wells located in a fluid line to sensing probes such that temperature and conductivity can be determined; transferring conductivity signals through at least 3 leads from the sensing probes; determining conductivity for each sensing probe; calculating the difference of conductivity from each sensing probe; and determining if the difference exceeds a threshold.

In accordance with another aspect of the invention there is provided apparatus comprising a fluid conduit in a cassette including a well for at least one of transmitting temperature and permitting conductivity sensing of fluid passing through the conduit, wherein the well is adapted for interconnection with a sensor.

In various alternative embodiments, the apparatus may be configured so that a portion of the well comes into contact with fluid in the conduit or so that no portion of the well comes into contact with fluid in the conduit. The fluid conduit in the cassette may include plastic tubing or metal tubing.

In various embodiments, the cassette containing the fluid line comprises a rigid body overlaid on one or more sides with a flexible diaphragm. In various embodiments the flexible diaphragm cassette includes one or more pump chambers and/or one or more value stations. In various embodiments, one or more wells are positioned on the edge of the cassette. In certain of these embodiments, one or more wells are positioned on the bottom edge of the cassette.

In various embodiments, the cassette has a rigid front and/or back plate. One or more wells may be installed in the rigid cassette. Alternatively, one or more sensor leads may be installed in the rigid cassette. In various embodiments, the rigid cassette may contain one or more pod pumps.

The cassette and the well may be integrally formed from the same material.

Alternatively, the well may be coupled to the cassette, e.g., using at least one of press fit connection, flexible tabs, adhesive, ultrasonic weld, and a retaining plate and fastener. An o-ring may be disposed between the well and the fluid conduit. The o-ring may include one of a round cross-section, a square cross-section, and an X-shaped cross-section. The well may include a groove to receive a portion of the o-ring. A portion of the well in contact with the conduit may be flexible so as to deform the conduit and may include a plurality of cuts to provide such flexibility.

In accordance with another aspect of the invention there is provided a fluid pumping apparatus comprising at least one pump and a well for at least one of transmitting temperature and permitting conductivity sensing of fluid passing through the conduit, wherein the well is adapted for interconnection with a sensor. In various alternative embodiments, the at least one pump may include at least one pod pump and may include a pair of pod pumps. The at least one pump and the well may be integrated into a cassette.

In accordance with another aspect of the invention there is provided a sensing system comprising at least one sensing probe and at least one well installed in a cassette, the well in communication with the sensing probe for at least one of thermal sensing and conductivity sensing.

In accordance with another aspect of the invention there is provided a sensor manifold comprising a cassette and at least one sensing probe for at least one of thermal sensing and conductivity sensing. In various embodiments, the sensor manifold contains two or more fluid paths and two or more sensing probes for at least one of thermal sensing and conductivity sensing. In various embodiments, the sensor manifold is passive with respect to controlling the flow of the fluid in the fluid paths within the cassette. In such embodiments, the sensor manifold may be free from valves and pumping mechanisms. In various embodiments, the sensor manifold may comprise a cassette with a rigid front and/or back plate and a mid-plate. In various embodiments, the sensor manifold may comprise electrical circuits connected to the sensing probes. In certain of these embodiments, the sensor manifold may comprise a printed circuit board.

These aspects of the invention are not meant to be exclusive or comprehensive and other features, aspects, and advantages of the present invention are possible and will be readily apparent to those of ordinary skill in the art when read in conjunction with the following description, the appended claims, and the accompanying drawings.

### Brief Description of the Drawings

The foregoing features of the invention will be more readily understood by reference to the following detailed description, taken with reference to the accompanying drawings, wherein:
FIG. 1A and 1B are embodiments of the sensing apparatus where the thermal well is a continuous part of the fluid line;
FIG. 2A and 2B are embodiments of the sensing apparatus where the thermal well is a separate part from the fluid line;
FIG. 3A and 3B are embodiments of the sensing apparatus showing various lengths and widths of the thermal well;
FIG. 4 is a pictorial view of a thermal well according to one embodiment of the sensing apparatus;
FIG. 5 is a cross sectional view of an exemplary embodiment of the thermal well;
FIGS. 6A and 6B show section views of embodiments of thermal wells having variable wall thickness;
FIGS. 7A-7S are sectional views of various embodiments of the thermal well embedded in a fluid line;
FIG. 8 is a section side view of one embodiment of the sensing probe;
FIG. 9 is an exploded view of the embodiment shown in FIG. 8;
FIG. 10 is a sectional view of an alternate embodiment of the tip of the sensing probe;
FIG. 11A is an alternate embodiment of the sensing probe;
FIG. 11B is an alternate embodiment of the sensing probe;
FIG. 12 is a side view of an alternate embodiment of the sensing probe;
FIG. 13A is a section view of a sensing probe coupled to a thermal well;
FIG. 13B is an alternate embodiment of the sensing probe shown in FIG. 13A;
FIG. 14A is a section view of a sensing probe as shown in FIG. 8 coupled to a thermal well;
FIG. 14B is an alternate embodiment of the sensing probe shown in FIG. 14A;
FIG. 15 is a sectional view of one exemplary embodiment of the sensor apparatus;
FIG. 16 shows an alternate embodiment of a sensing probe coupled to a thermal well;
FIG. 17 is a section view of one embodiment of a sensing probe coupled to a thermal well and suspended by a spring;
FIG. 18 is a section view of one embodiment of a sensing probe in a housing;
FIG. 19 is a section view of one embodiment of a sensing probe in a housing;
FIG. 20 is a section view of one embodiment of a sensing probe in a housing;
FIG. 21 is a side view of a fluid line including two sensors;
FIG. 22 is a section view of a fluid line with a sensor apparatus;
FIG. 23 A is a section view of the back side of an exemplary cassette;
FIG. 23B is a side view of the side of an exemplary cassette;
FIG. 23C is a section view of the front of an exemplary cassette;
FIG. 24 is a view of an exemplary cassette and thermal wells;
FIG. 25 is a view of an exemplary cassette with thermal wells installed;
FIG. 26 is a view of the thermal wells extending into a fluid line of an exemplar cassette;
FIG. 27 is a close up certain features of FIG. 26;
FIG. 28 is a section view of one embodiment of a sensing probe coupled to a thermal well installed in a cassette and suspended by a spring;
FIG. 29 is a sectional view of one embodiment of a pod-pump that is incorporated into embodiments of cassette;
FIGS. 30A are front and isometric views of the exemplary embodiment of the fluid side of the midplate of the cassette;
FIGS. 30B are front and isometric views of the exemplary embodiment of the air side of the midplate of the cassette;
FIGS. 31A are front and isometric views of the exemplary embodiment of the inner side of the bottom plate of the cassette;
FIGS. 31B are front and isometric views of the exemplary embodiment of the outer side of the bottom plate of the cassette;
FIG. 31C is a side view of the exemplary embodiment of the midplate plate of the cassette;
FIG. 32A is a top view of the assembled exemplary embodiment of the cassette;
FIG. 32B is a bottom view of the assembled exemplary embodiment of the cassette;
FIG. 32C is an exploded view of the assembled exemplary embodiment of the cassette;
FIG. 32D is an exploded view of the assembled exemplary embodiment of the cassette;
FIGS. 33A-33C show cross sectional views of the exemplary embodiment of the assembled cassette
FIG. 34 is a perspective view of a system having a base unit with a disposable unit containing a manifold according to one embodiment of the invention;
FIG. 35 is a perspective view of the disposable unit containing a manifold shown in FIG. 34;
FIG. 36A is a perspective view of the components from the system of FIG. 34;
FIG. 36B is a perspective, back-side cross-sectional view of the manifold of FIGS. 35 and 38A-B, in accordance with an exemplary embodiment of the present invention;
FIG. 36C shows a thermal well that may be used in the manifold of FIGS. 2, 49, and 13B in the heat-exchanger figure of FIG. 1, in accordance with an exemplary embodiment of the present invention;

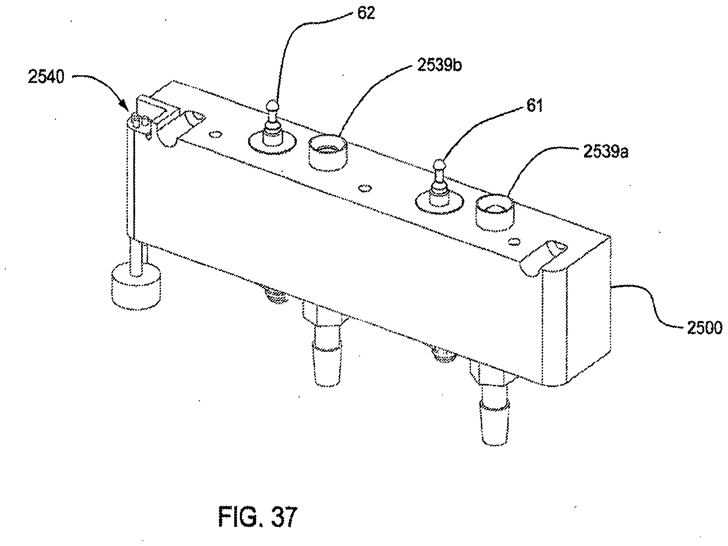

FIG. 37 shows a view of the manifold interface, in accordance with an exemplary embodiment of the present invention;
FIGS. 38A and 38B respectively show a perspective back-side view and a perspective bottom view of the manifold from FIG. 35, in accordance with an exemplary embodiment of the present invention;
FIG. 39 is a view of an exemplary sensor manifold; and
FIG. 40 is a view of another exemplary sensor manifold.
FIG. 41 is a view of another exemplary sensor manifold.
FIG. 42 is a view of the fluid paths within the exemplary sensor manifold shown in FIG. 41.
FIG. 43 is a side view of the exemplary sensor manifold shown in FIG. 41.
FIG. 44A is a cross sectional view of the exemplary sensor manifold shown in FIG. 41 at cross section A-A of FIG. 44B.
FIG. 44B is a front view of the exemplary sensor manifold shown in FIG. 41.
FIG. 45 is an exploded view of the exemplary sensor manifold shown in FIG. 41.
FIG. 46 is a view of a printed circuit board and media edge connector in accordance with the exemplary sensor manifold shown in FIG. 41.
FIG. 47 is an exemplary fluid schematic of a hemodialysis system.

It should be noted that the foregoing figures and the elements depicted therein are not necessarily drawn to consistent scale or to any scale. Unless the context otherwise suggests, like elements are indicated by like numerals.

### Detailed Description of Specific Embodiments

Definitions. As used in this description and the accompanying claims, the following terms shall have the meanings indicated, unless the context otherwise requires:
"Spheroid" means any three-dimensional shape that generally corresponds to a oval rotated about one of its principal axes, major or minor, and includes three-dimensional egg shapes, oblate and prolate spheroids, spheres, and substantially equivalent shapes.
"Hemispheroid" means any three-dimensional shape that generally corresponds to approximately half a spheroid.
"Spherical" means generally spherical.
"Hemispherical" means generally hemispherical.
"Fluid" shall mean a substance, a liquid for example, that is capable of being pumped through a flow line. Blood is a specific example of a fluid.
A "patient" includes a person or animal from whom, or to whom, fluid is pumped, whether as part of a medical treatment or otherwise.
"Subject media" is any material, including any fluid, solid, liquid or gas, that is in contact directly with a sensing probe or indirectly via thermal wells, sensor extension pins, and other such devices for transferring information regarding one or more characteristics of such subject media to one or more sensors.

Various aspects of the present invention are described below with reference to various exemplary embodiments. It should be noted that headings are included for convenience and do not limit the present invention in any way.

Various embodiments of sensors, including thermal and/or conductivity sensors, are described. Such thermal/conductivity sensors can be used in a wide variety of applications and are by no means limited to thermal/conductivity measurements of fluids or to thermal/conductivity measurements in any particular context. Additionally, various embodiments of systems, devices, and methods for sensor interface, including direct sensor contact, sensor interface through the use of a thermal well, or otherwise with various disposable and reusable components are described. Such systems, devices, and methods for sensor interface can be used with a wide variety of sensors and in a wide variety of applications. Such systems, devices, and methods for sensor interface are by no means limited to use with the various sensor embodiments or for use in any particular context.

### 1. THERMAL WELLS

In one exemplary embodiment, a thermal well is used to accommodate a sensor probe, such as a temperature sensing probe. The thermal well comes into direct contact with a subject media (e.g., a liquid such as blood or dialysate) and the sensing probe does not. Based on heat transfer dictated in large part by the thermodynamic properties of the thermal well and sensing probe construction, the sensing probe can determine the properties of the subject media without coming into direct contact with the subject media. The accuracy and efficiency of the sensor apparatus arrangement depends on many factors including, but not limited to: construction, material and geometry of both the probe and the thermal well.

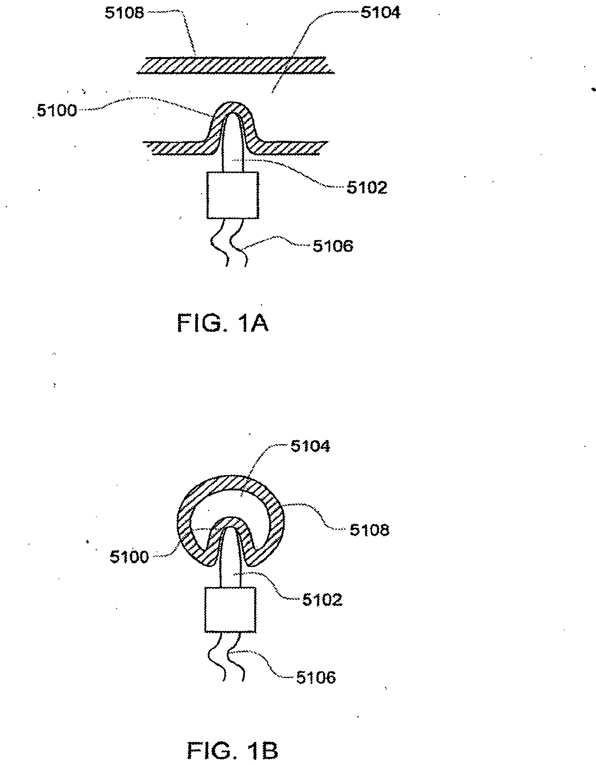

Referring now to FIGS. 1A and 1B, two embodiments of the sensor apparatus which includes the thermal well **5100** and the sensing probe **5102,** are shown in relation to a fluid line **5108.** In these embodiments, the thermal well **5100** is integrated into the fluid line **5108.** However, in other embodiment, some described below, the thermal well **5100** is not completely integrated into the fluid line **5108,** i.e., the thermal well **5100** can be made from different materials as compared with the fluid line **5108.** In alternate embodiments, the thermal well **5100** is not integrated into any fluid line but can be integrated into anything or nothing at all. For example, in some embodiments, the thermal well **5100** can be integrated into a container, chamber, machine, protective sleeve, fluid pump, pump cassette, disposable unit, manifold, or other assembly, sub-assembly, or component. For purposes of the description, an exemplary embodiment is described for illustrative purposes. The exemplary embodiment includes the embodiment where the thermal well **5100** is in a fluid line. However, the sensor apparatus and the thermal well can be used outside of a fluid line.

Referring now to FIG. 1A, a side view showing a thermal well **5100** formed in a fluid line **5108** which provides the space **5104** for subject media to flow through, and a sensing probe **5102** is shown. Data from the sensing probe is transmitted using at least one lead **5106.** An end view of FIG. 1A is shown in FIG. 1B.

In this embodiment, the thermal well **5100** is one piece with the fluid line **5108.** The total area of the thermal well **5100** can vary. By varying the geometry of the thermal well **5100,** the variables, including, but not limited to, the thermal conductivity characteristic of the thermal well **5100** and thus, the heat transfer between the thermal well **5100** and the sensing probe **5102** will vary. As described in more detail below, the material construction of the thermal well **5100** is another variable in the sensor apparatus.

In some embodiments, the fluid line **5108** is made from a material having a desired thermal conductivity. This material may vary depending on the purpose. The material can be anything including, but not limited to, any plastic, ceramic, metals or alloys of metals or combinations thereof.

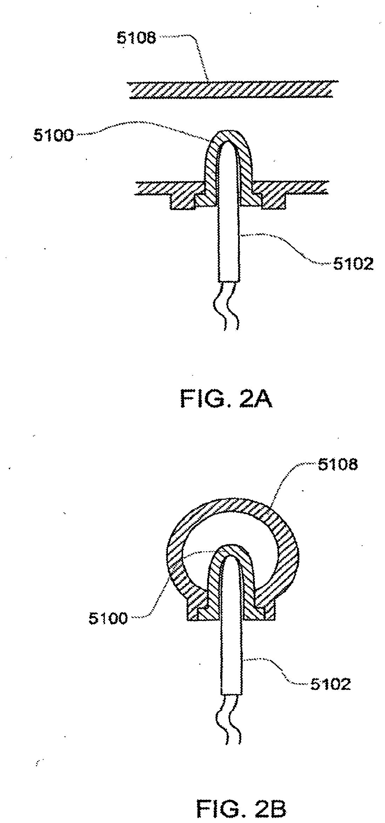

Referring now to FIGS. 2A and 2B, in these embodiments, the fluid line **5108** and the thermal well **5100** are separate parts. In some embodiments, the fluid line **5108** and the thermal well **5100** are made from different materials.

FIGS. 1A-1B and FIGS. 2A-2B show relatively simple embodiments of the sensor apparatus. Thus, for these embodiments, the sensing apparatus includes a thermal well **5100** and a sensing probe **5102** where the thermal well either is integrated as one continuous part with the fluid line **5108** or is a separate part from the fluid line **5108.** However, many embodiments of the sensor apparatus are contemplated. Much of the various embodiments include variations on the materials and the geometries of the thermal well **5100** and/or the sensing probe **5102.** These variations are dictated by multiple variables related to the intended use for the sensor apparatus. Thus, the subject media and the constraints of the desired sensor, for example, the accuracy, time for results and the fluid flow and subject media characteristics are but a sampling of the various constraints that dictate the embodiment used. In most instances, each of the variables will affect at least one part of the embodiment of the sensor apparatus.

Thus, multiple variables affect the various embodiments of the sensor apparatus, these variables include but are not limited to: 1) geometry of the thermal well; 2) material composition of the thermal well; 3) material composition of the sensing probe; 4) desired flow rate of the subject media; 5) length and width of the thermal well; 6) desired accuracy of the sensing probe; 7) wall thicknesses; 8) length and width of the sensing probe; 9) cost of manufacture; 10) subject media composition and characteristics including tolerance for turbulence; 11) geometry of sensing probe; and 12) desired speed of readings.

In the foregoing, various embodiments of the sensor apparatus are described. The description is intended to provide information on the affect the variables have on the sensor apparatus embodiment design. However, these are but exemplary embodiments. Many additional embodiments are contemplated and can be easily designed based on the intended use of the sensor apparatus. Thus, by changing one or more of the above mentioned partial list of variables, the embodiment of the sensor apparatus may vary.

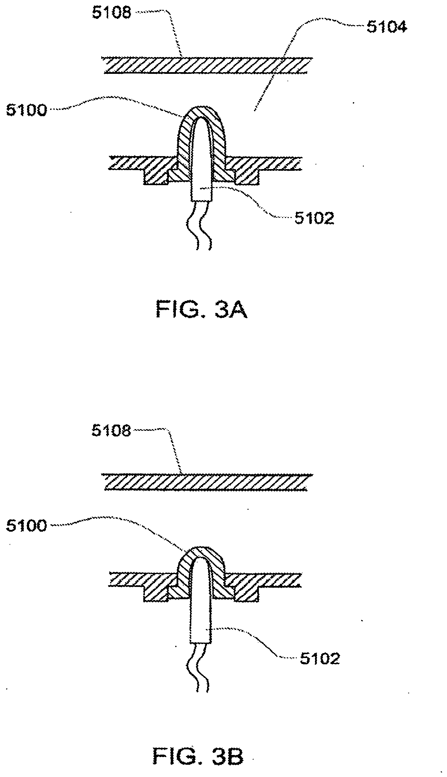

Referring now to FIGS. 3A and 3B, two embodiments of the thermal well **5100** are shown as different parts from the fluid line **5108.** These embodiments show two geometries of the thermal well **5100.** In FIG. 3A, the geometry includes a longer thermal well **5100.** In FIG. 3B, the thermal well **5100** geometry is shorter. The length and width of the thermal well **5100** produce varying properties and accuracies of the thermal conductivity between the thermal well **5100** and the sensing probe **5102.** Depending on the use of the sensor apparatus, the thermal well **5100** geometry is one variable.

Referring now to FIG. 3A, the longer thermal well **5100** generally provides a greater isolation between the subject media temperature in the fluid line **5104** and the ambient temperature. Although the longer thermal well **5100** geometry shown in FIG. 3A may be more accurate, the embodiment shown in FIG. 3B may be accurate enough for the purpose at hand. Thus, the length and width of the thermal well **5100** can be any length and width having the desired or tolerable accuracy characteristics. It should be understood that two extremes of length are shown in these embodiments; however, any length is contemplated. The description herein is meant to explain some of the effects of the variables.

Still referring to FIGS. 3A and 3B, the longer thermal well **5100** shown in FIG. 3A may impact the fluid flow of the subject media in the fluid line **5108** to a greater degree than the embodiment shown in FIG. 3B. It should be understood that the length of the thermal well **5100** may also impact the turbulence of the fluid flow. Thus, the length and width of the thermal well **5100** may be changed to have greater or lesser impact on the fluid flow and turbulence of the fluid, while mitigating the other variables.

The shape of the thermal well **5100** is also a variable. Any shape desired is contemplated. However, the shape of the thermal well **5100,** as with the other variables, is determined in part based on the intended use of the sensor apparatus. For purposes of description, an exemplary embodiment is described herein. However, the shape in the exemplary embodiment is not meant to be limiting.

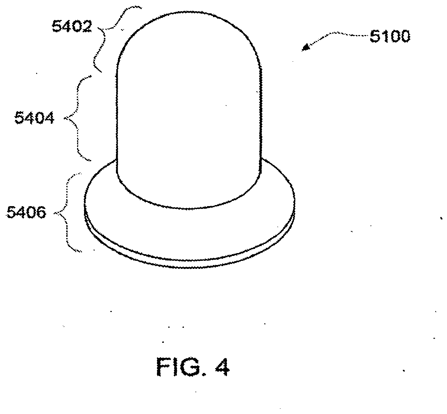

Referring now FIG. 4 for purposes of description, the thermal well **5100** has been divided into 3 zones. The top zone **5402** communicates with the sensing probe (not shown); the middle zone **5404** provides the desired length of the thermal well **5100.** As described above, the length may dictate the level of protrusion into the fluid path. The length is dictated in part by the desired performance characteristics as discussed above. The middle zone **5404** also isolates the top zone **5402** from the ambient. The middle zone **5404** may also serve to locate, fasten or seal the thermal well **5100** into the fluid line (shown as **5108** in FIGS. 1A-1B).

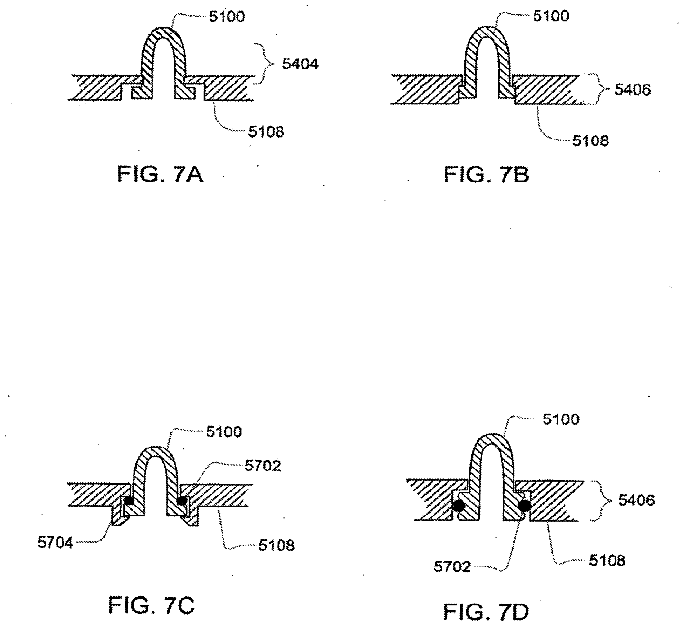

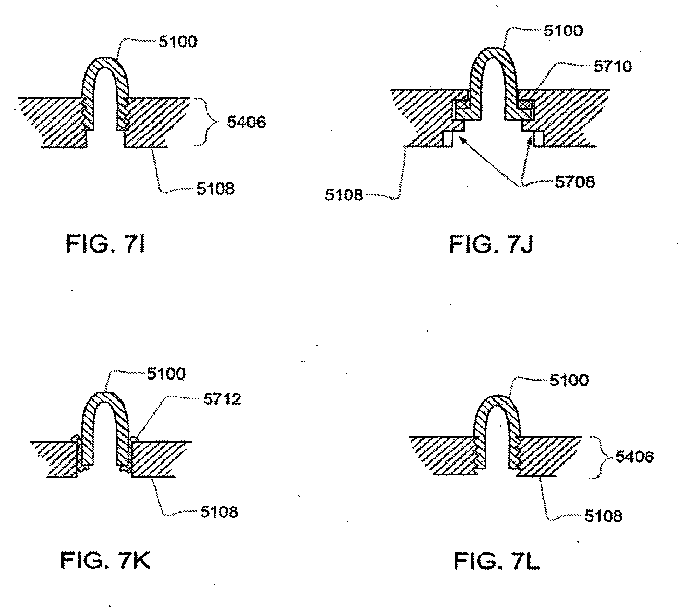

The bottom zone **5406,** which in some embodiments may not be necessary (see FIG. 7K) thus, in these embodiments, the middle zone **5404** and the bottom zone **5406** may be a single zone. However, in the exemplary embodiment, the bottom zone **5406** is shaped to aid in press fitting the thermal well into an area in the fluid line and may locate and/or fasten the thermal well **5100** into the fluid line **5108.** In other embodiments, zone **5406** may be formed to facilitate various joining methods (see FIGS. 7A-7J, 7L-7S)

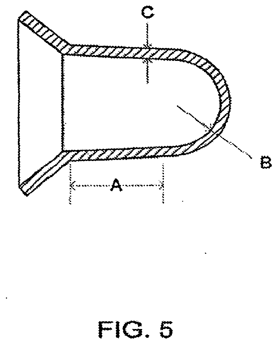

Referring now to FIG. 5 a cross section of the exemplary embodiment of the thermal well **5100** is shown. The dimensions of the exemplary embodiment of the thermal well **5100** include a length A of approximately .113 inches (with a range from 0-.379 inches), a radius B of approximately .066 inches and a wall thickness C ranging from approximately .003-.009 inches. These dimensions are given for purposes of an exemplary embodiment only. Depending on the variables and the intended use of the sensing apparatus, the thermal well **5100** dimensions may vary, and the various embodiments are not necessarily proportional.

In some embodiments, the wall thickness can be variable, i.e., the wall thickness varies in different locations of the thermal well. Although these embodiments are shown with variable thicknesses in various locations, this is for description purposes only. Various embodiments of the thermal well may incorporate varying wall thickness in response to variables, these varying wall thicknesses can be "mixed and matched" depending on the desired properties of the sensing apparatus. Thus, for example, in some embodiments, a thinner zone **5404** may be used with thinner zone **5406** and vice-versa. Or, any other combination of "thinner" and "thicker" may be used. Also, the terms used to describe the wall thicknesses are relative. Any thickness desired is contemplated. The figures shown are therefore for descriptive purposes and represent two embodiments where many more are contemplated.

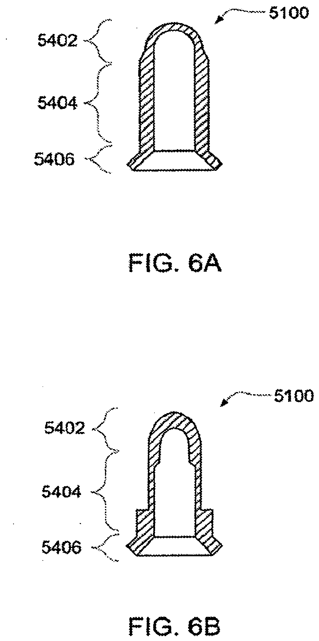

Referring now to FIGS. 6A and 6B, zone **5402** can be thicker or thinner as desired. The thinner zone **5402,** amongst other variables, generally provides for a faster sensing time while a thicker zone may be useful for harsh environments or where sensor damping is desired. Zone **5404** may be thicker, amongst other variables, for greater strength or thinner for, amongst other variables, greater isolation from ambient. Zone **5406** can be thinner or thicker depending on the fastening method used.

The thermal well **5100,** in practice, can be embedded into a fluid line **5108,** as a separate part from the fluid line **5108.** This is shown and described above with respect to FIGS. 2A-2B. Various embodiments may be used for embedding the thermal well **5100** into the fluid line **5108.** Although the preferred embodiments are described here, any method or process for embedding a thermal well **5100** into a fluid line **5108** can be used. Referring now to FIGS. 7A-7S, various configurations for embedding the thermal well **5100** into the fluid line **5108** are shown. For these embodiments, the thermal well **5100** can be made from any materials, including but not limited to, plastic, metal, ceramic or a combination thereof. The material may depend in some part on the compatibility with the intended subject media. The fluid line **5108,** in these embodiments, may be made from plastic, metal, or any other material that is compatible with the subject media.

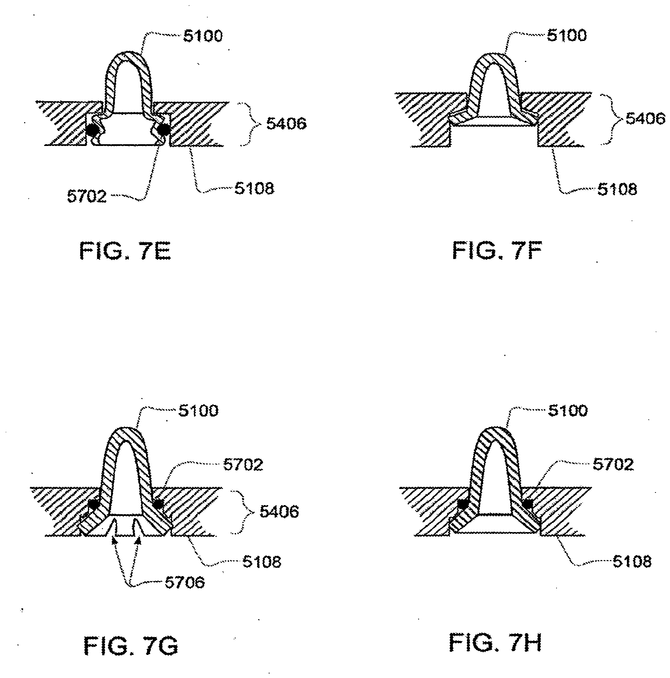

Referring first to FIG. 7A, the thermal well **5100** is shown press fit into the fluid line **5108** using the zone **5404** (shown in FIG. 4). In FIG. 7B, the thermal well **5100** is shown press fit into the fluid line **5108** using the zone **5406.** Referring now to FIG. 7C, the thermal well **5100** is shown retained in the fluid line **5108** with flexible tabs **5704,** an O-ring is also provided. Referring now to FIG. 7D, the thermal well **5100** is shown inserted into the fluid line **5108** with an O-ring **5702.** The thermal well **5100** is also shown as an alternate embodiment, where the thermal well **5100** zone **5406** includes an O-ring groove. The O-ring groove can be cut, formed, spun, cast or injection molded into the thermal well, or formed into the thermal well **5100** by any other method. FIG. 7E shows a similar embodiment to that shown in FIG. 7D, however, the O-ring groove is formed in zone **5406** rather than cut, molded or cast as shown in FIG. 7D.

Referring now to FIG. 7F, the thermal well **5100** is shown press fit into the fluid line **5108,** zone **5406** includes flexibility allowing the edge of zone **5406** to deform the material of the fluid line **5108.** Referring now to FIG. 7G, the thermal well **5100** includes cuts **5706** on the zone **5406** providing flexibility of the zone **5406** for assembly with the fluid line **5108.** An O-ring **5702** is also provided. Although two cuts are shown, a greater number or fewer cuts are used in alternate embodiments.

Referring now to FIG. 7H, the embodiment shown in FIG. 7F is shown with the addition of an O-ring **5702.** Referring to FIG. 7I, the thermal well **5100** is shown insert molded in the fluid line **5108.** Zone **5406** is formed to facilitate or enable assembly by insert molding.

FIG. 7J shows an embodiment where the thermal well **5100** is heat staked **5708** to retain the thermal well **5100** in the fluid line **5108.** In some embodiments of FIG. 7J, an O-ring **5710** is also included. In this embodiment, the O-ring **5710** has a rectangular cross section. However, in alternate embodiments, the O-ring may have a round or X-shaped cross section. Likewise, in the various embodiments described herein having an O-ring, the O-ring in those embodiments can have a round, rectangular or X-shaped cross section, or any cross sectional shape desired.

Referring now to FIG. 7K, the thermal well **5100** is retained in the fluid line **5108** by adhesive **5712.** The adhesive can be any adhesive, but in one embodiment, the adhesive is a UV curing adhesive. In alternate embodiments, the adhesive may be any adhesive that is compatible with the subject media. In this embodiment, the thermal well **5100** is shown without a zone **5406.**

Referring now to FIG. 7L, thermal well **5100** is shown ultrasonically welded in the fluid line **5108.** The zone **5406** is fabricated to enable joining by ultrasonic welding.

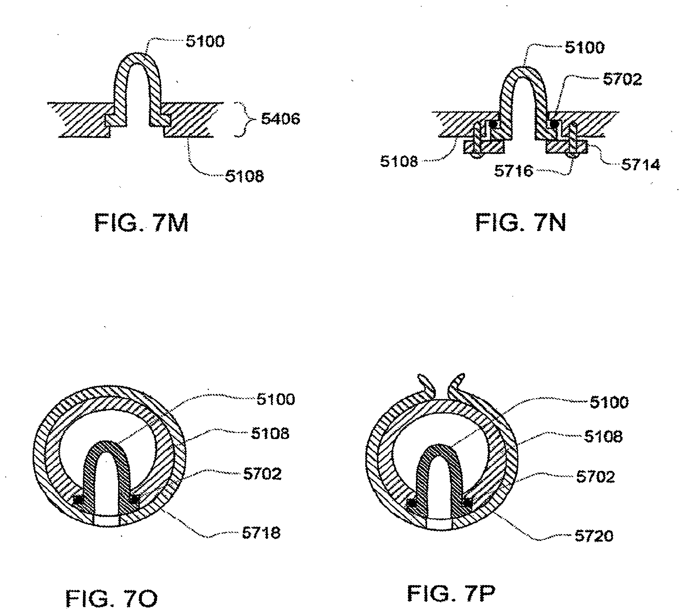

Referring now to FIG. 7M, a thermal well **5100** is shown insert molded in the fluid line **5108.** Zone **5406** is a flange for the plastic in the fluid line **5108** to flow around. In the embodiment shown, the flange is flat, however, in other embodiments; the flange may be bell shaped or otherwise.

Referring now to FIG. 7N, the thermal well **5100** is shown retained in the fluid line **5108** by a retaining plate **5714** and a fastener **5716.** O-ring **5702** is also shown.

Referring now to FIGS. 7O-7P, an end view is shown of a thermal well **5100** that is retained in a fluid line **5108** by a retaining ring **5718** (FIG. 7O) or in an alternate embodiment, a clip **5720** (FIG. 7P). O-ring **5702** is also shown.

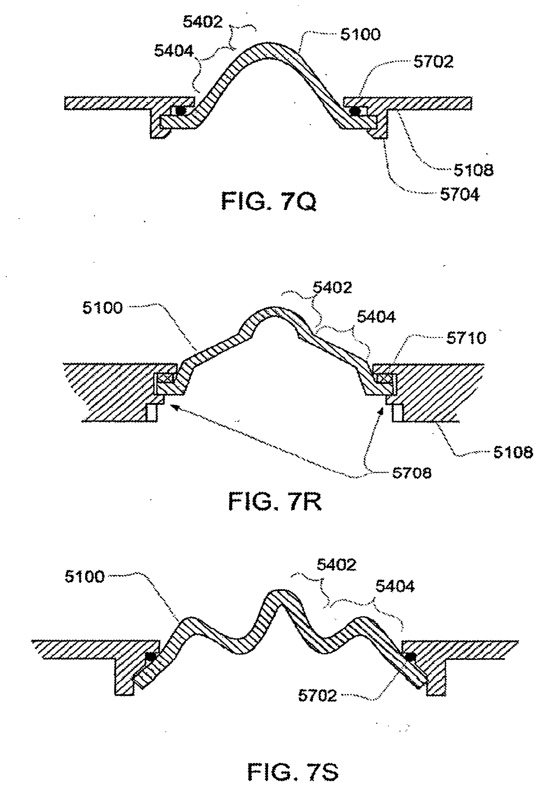

Referring now to FIG. 7Q, the embodiment of FIG. 7C is shown with an alternate embodiment of the thermal well **5100.** In this embodiment of the thermal well **5100** the referred to as zone **5404** in FIG. 4 includes a taper that may allow for easier alignment with a sensing probe, better isolation of zone **5402** from the ambient and better flow characteristics in the fluid path. The thermal well **5100** is shown retained in the fluid line **5108** using flexible tabs **5704.** An O-ring is also provided.

FIG. 7R shows the embodiment of FIG 7J with an alternate embodiment of the thermal well **5100.** The thermal well **5100** shown in this embodiment has a taper in zone **5404** that may allow for easier alignment with a sensing probe, may allow better isolation of zone **5402** from the ambient and may allow better flow characteristics in the fluid path. Zone **5402** provides a hemispherical contact for effective thermal coupling with a thermal probe. The thermal well **5100** is heat staked **5708** to retain the thermal well **5100** in the fluid line **5108.** In some embodiments of FIG. 7R, an O-ring **5710** is also included. In this embodiment, the O-ring **5710** has a rectangular cross section. However, in alternate embodiments, the O-ring can have a round or X-shaped cross section.

Referring now to FIG. 7S, the embodiment of FIG. 7H is shown with an alternate embodiment of the thermal well **5100.** FIG. 7S is shown with the addition of an O-ring **5702.** In this embodiment of the thermal well **5100** zone **5404** (as shown in FIG. 4) has convolutions that may allow better isolation of zone **5402** from the ambient. While several geometries have been shown for zone **5404,** many others could be shown to achieve desired performance characteristics.

### 2. SENSING PROBES

Various embodiments of systems, devices, and methods for sensor interface, including direct sensor contact, sensor interface through the use of a thermal well, or otherwise with various disposable and reusable components are described. Such systems, devices, and methods for sensor interface can be used with a wide variety of sensors and in a wide variety of applications. Such systems, devices, and methods for sensor interface are by no means limited to use with the various sensor embodiments or for use in any particular context.

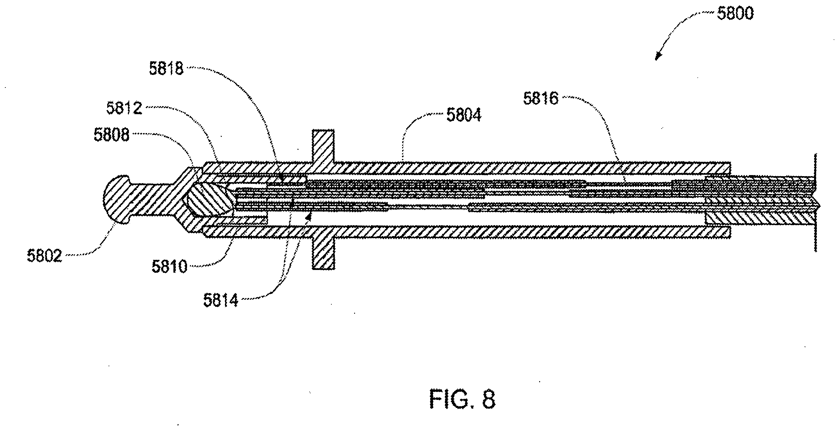

Referring now to FIG. 8, a sectional view of an exemplary embodiment of a sensing probe **5800** is shown. The housing **5804** is a hollow structure that attaches to the tip **5802.** The tip is made of a highly thermally conductive material. The housing **5804,** in the exemplary embodiment, is made from a thermally insulative material. In some embodiments, the housing is made of a thermally and electrically insulative material. In the exemplary embodiment, the housing **5804** is made of plastic which is a thermally insulative and electrically insulative material. The tip **5802** either contacts the subject media directly, or else is mated with a thermal well.

In the exemplary embodiment, the tip **5802** is attached to the housing **5804** using a urethane resin or another thermal insulator in between (area **5807**) the tip **5802** and the housing **5804.** Urethane resin additionally adds structural support. In alternate embodiments, other fabrication and joining methods can be used to join the tip **5802** to the housing **5804.**

The tip **5802** of the sensing probe **5800** is made of a thermally conductive material. The better thermally conductive materials, for example, copper, silver and steel, can be used, however, depending on the desired use for the sensing probe and the subject media; the materials may be selected to be durable and compatible for the intended use. Additionally, factors such as cost and ease of manufacture may dictate a different material selection. In one exemplary embodiment, the tip **5802** is made from copper. In other embodiments, the material can be an alloy of copper or silver, or either solid or an alloy of any thermally conductive material or element, including but not limited to metals and ceramics. However, in the exemplary embodiments, the tip **5802** is made from metal.

In the exemplary embodiment, the tip **5802** is shaped to couple thermally with a thermal well as described in the exemplary embodiment of the thermal well above. In the exemplary embodiment as well as in other embodiments, the tip **5802** may be shaped to insulate the thermal sensor **5808** from the ambient. In the exemplary embodiment, the tip **5802** is made from metal.

In alternate embodiments a non-electrically conductive material is used for the tip. These embodiments may be preferred for use where it is necessary to electrically insulate the thermal well from the probe. In another alternate embodiment, the tip 5802 may be made from any thermally conductive ceramic.

In the exemplary embodiment, the thermal sensor **5808** is located in the housing and is attached to the interior of the tip **5802** with a thermally conductive epoxy **5812.** In the exemplary embodiment, the epoxy used is THERMALBOND, however, in other embodiments; any thermal grade epoxy can be used. However, in alternate embodiments, thermal grease may be used. In alternate embodiments, an epoxy or grease is not used.

The thermal sensor **5808,** in the exemplary embodiment, is a thermistor. The thermistor generally is a highly accurate embodiment. However in alternate embodiments, the thermal sensor **5808** can be a thermocouple or any other temperature sensing device. The choice of thermal sensor **5808** may again relate to the intended use of the sensing apparatus.

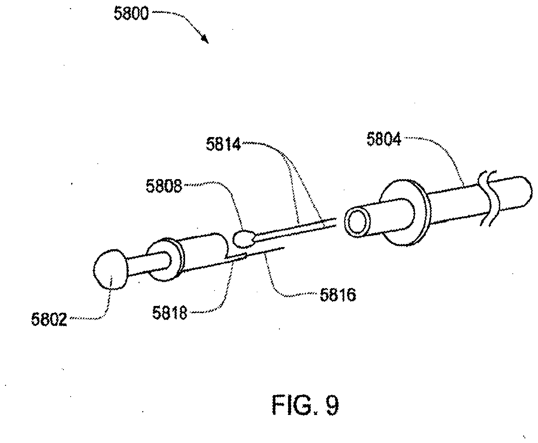

Leads **5814** from the thermal sensor **5808** exit the back of the housing **5804.** These leads **5814** attach to other equipment used for calculations. In the exemplary embodiment, a third lead **5816** from the tip **5802** is also included. This third lead **5816** is attached to the tip on a tab **5818.** The third lead **5816** is attached to the tip **5802** because in this embodiment, the tip **5802** is metal and the housing is plastic. In alternate embodiments, the housing **5804** is metal, thus the third lead **5816** may be attached to the housing **5804.** Thus, the tip **5802,** in the exemplary embodiment, includes a tab **5818** for attachment to a lead. However, in alternate embodiments, and perhaps depending on the intended use of the sensing apparatus, the third lead **5816** may not be included. Also, in alternate embodiments where a third lead is not desired, the tip **5802** may not include the tab **5818.** Referring now to FIG. 9, an exploded view of the sensing probe **5800** is shown.

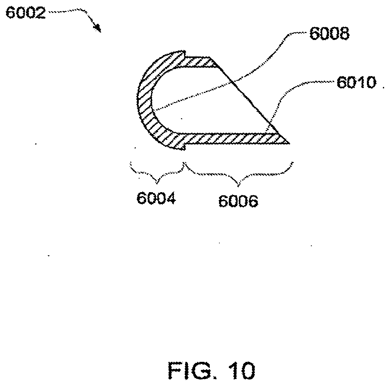

Referring now to FIG. 10 an alternate embodiment of the exemplary embodiment is shown. In this embodiment, the tip **6002** of the sensing probe is shown. The tip **6002** includes a zone **6004** that will contact either a subject media to be tested or a thermal well. A zone **6006** attaches to the sensor probe housing (not shown). An interior area **6008** accommodates the thermal sensor (not shown). In this embodiment, the tip **6002** is made from stainless steel. However, in other embodiments, the tip **6002** can be made from any thermally conductive material, including but not limited to: metals (including copper, silver, steel and stainless steel), ceramics or plastics.

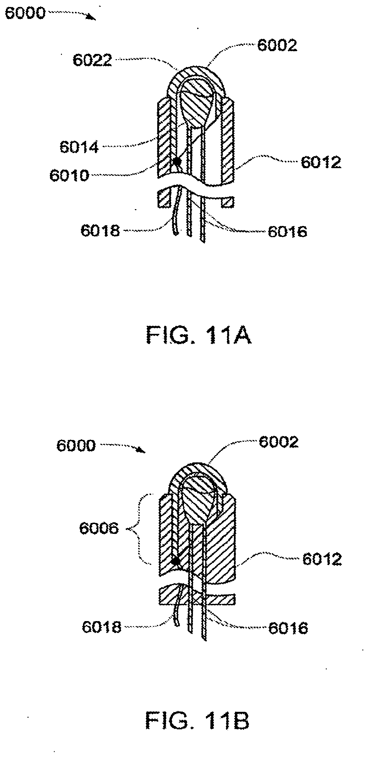

In the exemplary embodiment, zone **6006** includes a tab **6010.** A third lead (as described with respect to FIG. 8, **5816)** attaches from the tab **6010.** Referring next to FIGS. 11A and 11B, the sensing probe **6000** is shown including the tip **6002** and the housing **6012.** In one embodiment, the housing **6012** is made from any thermally insulative material, including but not limited to, plastic. In one embodiment, the housing **6012** is press fit to the tip **6002,** glued or attached by any other method. In one embodiment, the thermal sensor **6014** is thermally coupled to the tip **6002** with thermal grade epoxy or, in alternate embodiments, thermal grease **6022.** Two leads **6016** from the thermal sensor **6014** extend to the distal end of the housing. In some embodiments, a third lead **6018** is attached to the tip **6002** from the tab **6010.** As discussed above, in some embodiments where the third lead is not desired, the tip **6002** does not include a tab **6010.**

Referring now to FIG. 11B, an alternate embodiment of the sensing probe **6000** is shown. In this embodiment, the housing **6012** is a plastic molded over zone **6006** of the tip **6002** and the leads **6016,** and in some embodiments, a third lead **6018.**

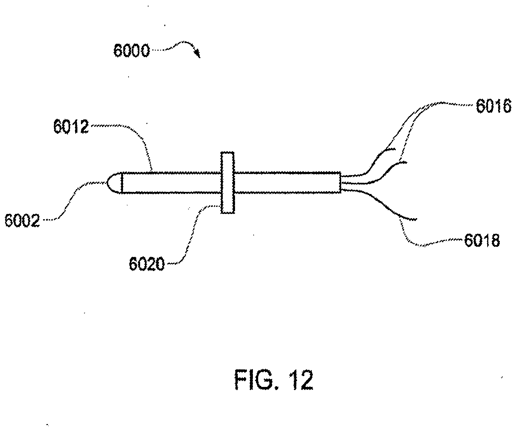

Referring now to FIG. 12, a full side view of one embodiment of the sensing probe **6000** shown in FIGS. 10-11B is shown. The sensing probe **6000** includes a housing **6012,** a tip **6002** and the leads **6016, 6018.** Flange **6020** is shown. In some embodiment, flange **6020** is used to mount and/or attachment to equipment.

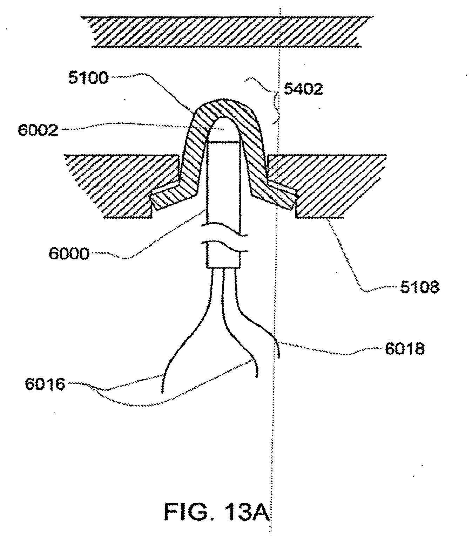

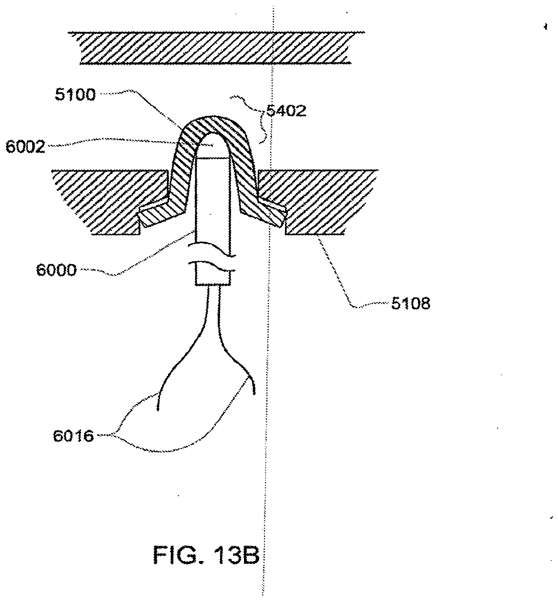

Referring now to FIG. 13A, the sensing probe **6000** shown in FIGS. 10-12, is shown coupled to a thermal well **5100** which is fastened into a fluid line **5108.** In the embodiment as shown, two leads **6016** are shown at the distal end of the sensing probe **6000.** And, in some embodiments, a third lead **6018** is also incorporated into the sensing probe **6000.** FIG. 13B shows an alternate embodiment where the sensing probe **6000** includes two leads **6016** but does not include the third lead **6018.**

Referring now to both FIGS. 13A and 13B, the tip **6002** of the sensing probe **6000** is in direct contact with the thermal well **5100.** Referring back to FIG. 4 and still referring to FIG. 13A and 13B the thermal well **5100** includes a zone **5402.** The thermal well **5100** is hollow, and the inner part of zone **5402** is formed such that it will be in mating contact with the sensing probe tip **6002.** As shown in this embodiment, the thermal well **5100** is designed to have a mating geometry with the sensing probe **6000.** Thus, the geometry of the thermal well **5100** may depend on the geometry of the tip **6002** of the sensing probe **6000** and vice-versa. In some embodiments, it may be desirable that the sensing probe **6000** does not have a tight fit or a perfect mate with the thermal well **5100.**

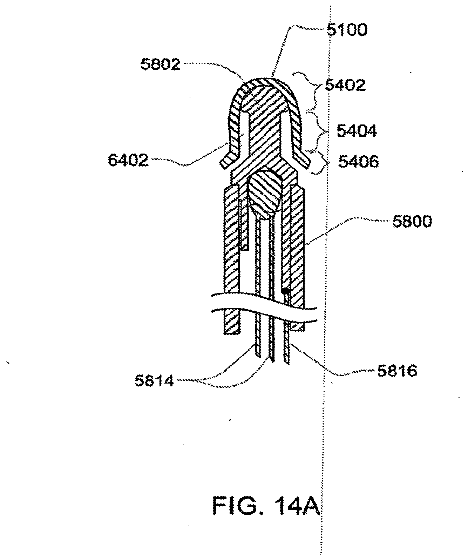

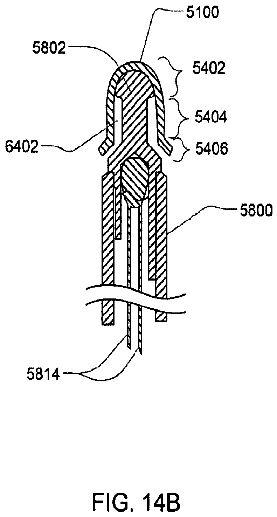

Referring now to FIG. 14A, one embodiment of the sensing probe **5800** (as shown in FIG. 8) is shown coupled to a thermal well **5100** which is fastened into a fluid line **5108.** In the embodiment as shown, two leads **5814** are shown at the distal end of the sensing probe **5800.** In some embodiments, a third lead **5816** is also incorporated into the sensing probe **5800.** FIG. 14B shows an alternate embodiment where the sensing probe **5800** includes two leads **5814** but does not include the third lead **5816.**

Referring now to both FIGS. 14A and 14B, the tip **5802** of the sensing probe **5800** is in direct contact with the thermal well **5100.** Referring back to FIG. 4 and still referring to FIG. 14A and 14B, the thermal well **5100** includes a zone **5402.** The thermal well **5100** is hollow, and the inner part of zone **5402** is formed such that it will be in mating contact with the sensing probe tip **5802.** As shown in this embodiment, the thermal well **5100** is designed to have a mating geometry with the sensing probe **5800.** Thus, the geometry of the thermal well **5100** depends on the geometry of the tip **5802** of the sensing probe **5800** and vice-versa.

### 3. SENSOR APPARATUS AND SENSOR APPARATUS SYSTEMS

### 3.1. SENSOR APPARATUS AND SENSOR APPARATUS SYSTEMS UTILIZED IN CONNECTION WITH A FLUID LINE

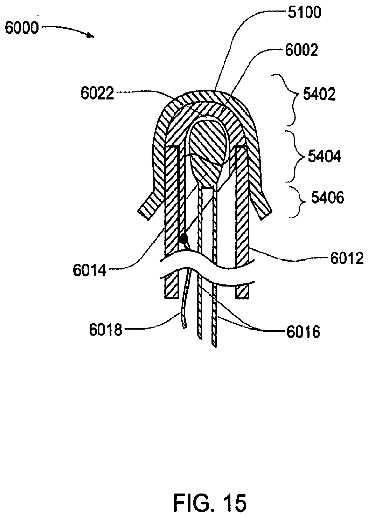

For purposes of description of the sensor apparatus, the sensor apparatus is described with respect to exemplary embodiments. The exemplary embodiments are shown in FIGS. 13A, 13B, and FIG. 15, with alternate exemplary embodiments in 14A and 14B. In alternate embodiments of the sensor apparatus, the sensing probe can be used outside of the thermal well. However, the sensor apparatus has already been described herein alone. Thus, the description that follows describes one embodiment of the exemplary embodiment of the sensor apparatus which includes, for this purpose, a sensing probe and a thermal well.

Referring now to FIG. 15, in an exemplary embodiment, the sensing probe **6000** shown in FIG. 13A and the thermal well **5100** are shown coupled and outside of a fluid line. As described above, the thermal well **5100** can be in a fluid line, a protective sleeve, any disposable, machine, chamber, cassette or container. However, for purposes of this description of the exemplary embodiment, the thermal well **5100** is taken to be anywhere where it is used to determine thermal and/or conductive properties (FIG. 13A) of a subject media.

A subject media is in contact with the outside of zone **5402** of the thermal well **5100.** Thermal energy is transferred from the subject media to the thermal well **5100** and further transferred to the tip **6002** of the sensing probe **6000.** Thermal energy is then conducted to the thermal sensor **6014.** The thermal sensor **6014** communicates via leads **6016** with equipment that can determine the temperature of the subject media based on feedback of the thermal sensor **6014.** In embodiments where conductivity sensing is also desired, lead **6018** communicates with equipment that can determine the conductivity of the subject media. With respect to determining the conductivity of the subject media, in addition to the lead **6018,** a second electrical lead/contact (not shown) would also be used. The second lead could be a second sensor apparatus as shown in FIG. 15, or, alternatively, a second probe that is not necessarily the same as the sensor apparatus shown in FIG. 15, but rather, any probe or apparatus capable of sensing capacitance of the subject media, including, an electrical contact.

Heat transfer from the tip **6002** to the thermal sensor **6014** may be improved by the use of a thermal epoxy or thermal grease **6022.**

Referring now to FIGS. 14A and 14B, in the alternate exemplary embodiment, whilst the sensing probe **5800** is coupled to the thermal well **5100,** the tip **5802,** having the geometry shown, forms an air gap **6402** between the inner zones **5404** and **5406** of the thermal well **5100** and the tip **5802.** The air gap **6402** provides an insulative barrier so that only the top of the sensing tip of **5802** is in communication with the top zone **5402** of the thermal well **5100.**

The sensing probe **5800** and thermal well **5100** are shown coupled and outside of a fluid line. As described above, the thermal well **5100** can be in a fluid line, a protective sleeve, disposable unit, machine, non-disposable unit, chamber, cassette or container. However, for purposes of this description of the exemplary embodiment, the thermal well **5100** is taken to be anywhere where it is used to determine thermal and/or conductive properties (FIG. 14A) of a subject media.

A subject media is in contact with the outside of zone **5402** of the thermal well **5100.** Thermal energy is transferred from the subject media to the thermal well **5100** and further transferred to the tip **5802** of the sensing probe **5800.** Thermal energy is then conducted to the thermal sensor **5808.** The thermal sensor **5808** communicates via leads **5814** with equipment that can determine the temperature of the subject media based on feedback of the thermal sensor **5808.** In embodiments where conductivity sensing is also desired, lead **5816** communicates with equipment that can determine the conductivity of the subject media. With respect to determining the conductivity of the subject media, in addition to the lead **5816,** a second electrical lead (not shown) would also be used. The second lead could be a second sensor apparatus as shown in FIG. 14A, or, alternatively, a second probe that is not necessarily the same as the sensor apparatus shown in FIG. 14A, but rather, any probe or apparatus capable of sensing capacitance of the subject media, including, an electrical contact.

Heat transfer from the tip **5802** to the thermal sensor **5808** can be improved by the use of a thermal epoxy or thermal grease **5812.**

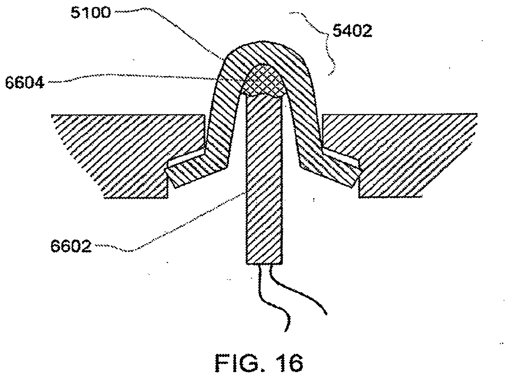

Referring now to FIG. 16, an alternate embodiment showing a sensing probe **6602** coupled to a thermal well **5100** is shown. For purposes of this description, any embodiment of the sensing probe **6602** and any embodiment of the thermal well **5100** can be used. In this embodiment, to increase the thermal coupling between the tip of the sensing probe **6602** and the thermal well **5100,** thermal grease **6604** is present at the interface of the tip of the sensing probe **6602** and the inner zone **5402** of the thermal well **5100.** In one embodiment, the amount of thermal grease **6604** is a volume sufficient to only be present in zone **5402.** However, in alternate embodiments, larger or smaller volumes of thermal grease can be used.

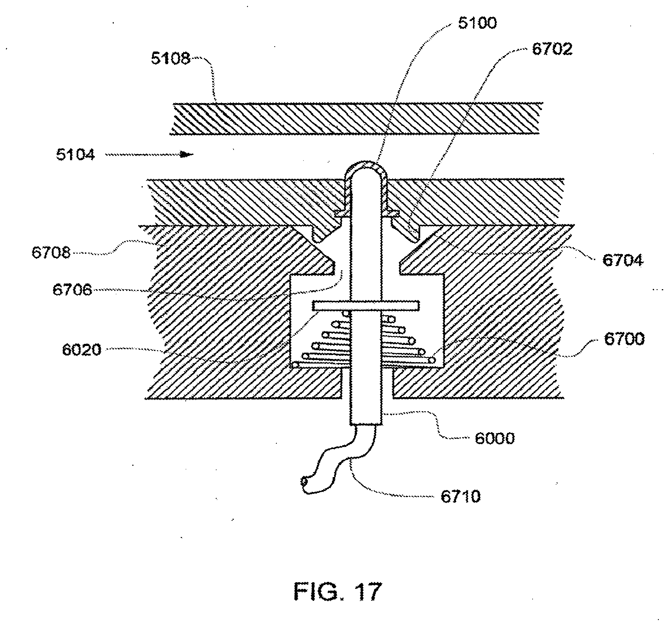

Referring now to FIG. 17, a sensor apparatus system is shown. In the system, the sensor apparatus is shown in a device containing a fluid line **5108.** The sensor apparatus includes the sensing probe **6000** and the thermal well **5100.** In this embodiment, the thermal well **5100** and fluid line **5108** is a disposable portion and the sensing probe **6000** is a reusable portion. Also in the reusable portion is a spring **6700.** The spring **6700** and sensing probe **6000** are located in a housing **6708.** The housing **6708** can be in any machine, container, device or otherwise. The spring **6700** can be a conical, a coil spring, wave spring, or urethane spring.

In this embodiment, the thermal well **5100** and the sensing probe **6000** may include alignment features **6702, 6704** that aid in the thermal well **5100** and sensing probe **6000** being aligned. The correct orientation of the thermal well **5100** and the sensing probe **6000** may aid in the mating of the thermal well **5100** and the sensing probe **6000** to occur. The configuration of the space **6706** provides the sensing probe **6000** with space for lateral movement. This allows the sensing probe **6000** to, if necessary; move laterally in order to align with the thermal well **5100** for mating.

The sensing probe **6000** is suspended by a spring **6700** supported by the flange **6020.** The spring **6700** allow vertical movement of the sensing probe **6000** when the thermal well **5100** mates with the sensing probe **6000.** The spring **6700** aids in establishing full contact of the sensing probe **6000** and the thermal well **5100.**

The fluid line **5108** can be in any machine, container, device or otherwise. The fluid line **5108** contains a fluid path **5104.** A subject media flows through the fluid path **5104** and the thermal well **5100,** located in the fluid line **5108** such that the thermal well **5100** has ample contact with the fluid path **5104** and can sense the temperature properties and, in some embodiments, the conductive properties of the subject media. The location of the thermal well **5100** in the fluid path **5104,** as described in more detail above, may be related to the desired accuracy, the subject media and other considerations.

The spring **6700** and sensing probe **6000** assembly, together with the space **6706** in the housing **6708** may aid in alignment for the mating of the sensing probe **6000** and the thermal well **5100.** The mating provides the thermal contact so that the thermal well **5100** and the sensing probe **6000** are thermally coupled.

A wire **6710** is shown. The wire contains the leads. In some embodiments, there are two leads. Some of these embodiments are temperature sensing. In other embodiments, the wire contains three or more leads. Some of these embodiments are for temperature and conductivity sensing.

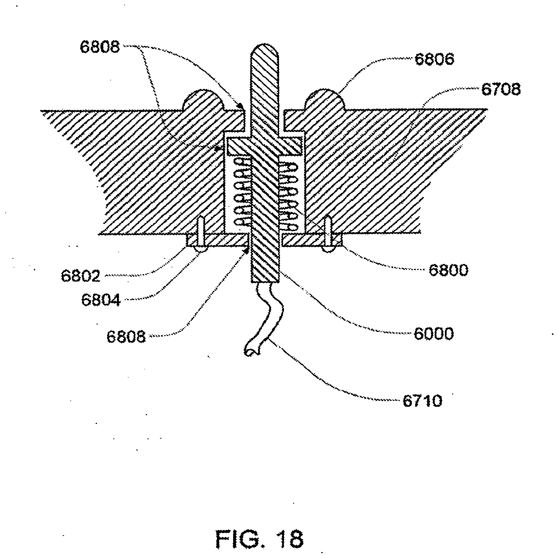

Referring now to FIG. 18, an alternate embodiment of the system shown in FIG. 17 is shown. In this embodiment, the sensing probe **6000** is suspended by a coil spring **6800.** A retaining plate **6802** captures the coil spring **6800** to retain the spring **6800** and sensing probe **6000.** In one embodiment, the retaining plate **6802** is attached to the housing **6708** using screws. However, in alternate embodiments, the retaining plate **6802** is attached to the housing **6708** using any fastening method including but not limited to: adhesive, flexible tabs, press fit, and ultrasonic welding. Aligning features **6806** on the housing **6708** aid in alignment of the sensing probe **6000** to a thermal well (not shown). Lateral movement of the sensing probe **6000** is provided for by clearance in areas **6808** in the housing **6708.** A wire **6710** is shown. The wire contains the leads. In some embodiments, there are two leads. Some of these embodiments are temperature sensing. In other embodiments, the wire contains three or more leads. Some of these embodiments are for temperature and conductivity sensing.

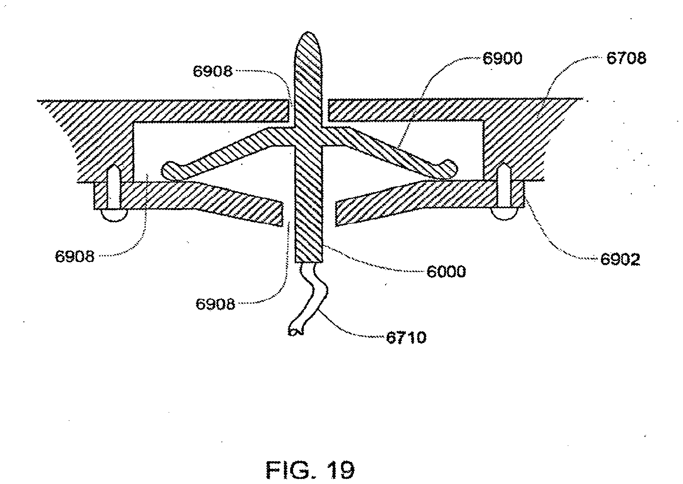

Referring now to FIG. 19, a sensing probe **6000** is shown in a housing **6708.** In these embodiments, an alternate embodiment of a spring, a flexible member **6900,** is integrated with the sensing probe **6000** to allow vertical movement of the sensing probe **6000** within the housing **6708.** A retaining plate **6902** captures the flexible member **6900** to retain the flexible member **6900** and sensing probe **6000.** In one embodiment, the retaining plate **6902** is attached to the housing **6708** using screws. However, in alternate embodiments, the retaining plate **6902** is attached to the housing **6708** using any fastening method including but not limited to: adhesive, flexible tabs, press fit, and ultrasonic welding. Lateral movement of the sensing probe **6000** is provided for by clearance in areas **6908** in the housing **6708.** A wire **6710** is shown. The wire contains the leads. In some embodiments, there are two leads. Some of these embodiments are temperature sensing. In other embodiments, the wire contains three or more leads. Some of these embodiments are for temperature and conductivity sensing.

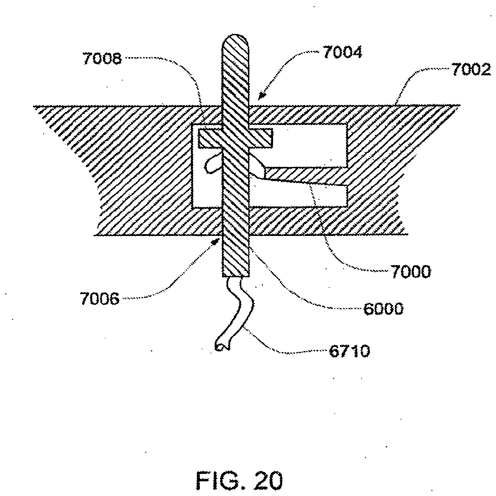

Referring now to FIG. 20, an alternate embodiment of a sensing probe **6000** in a housing **7002** is shown. In this embodiment, flexible member **7000** is attached or part of the housing **7002,** provides for vertical movement of the sensing probe **6000.** In this embodiment, the openings **7004, 7006** in housing **7002** are sized such that the sensing probe **6000** experiences limited lateral movement. Flexible member **7000** acts on the flange **7008** on the sensing probe **6000.** A wire **6710** is shown. The wire contains the leads. In some embodiments, there are two leads. Some of these embodiments are temperature sensing. In other embodiments, the wire contains three or more leads. Some of these embodiments are for temperature and conductivity sensing.

The flange, as shown and described with respect to FIGS. 12, 17, 20, can be located in any area desired on the sensing probe **6000.** In other embodiments, the sensing probe may be aligned and positioned by other housing configurations. Thus, the embodiments of the housing shown herein are only some embodiments of housings in which the sensor apparatus can be used. The sensor apparatus generally depends on being located amply with respect to the subject media. The configurations that accomplish this can vary depending on the subject media and the intended use of the sensing apparatus. Further, in some embodiments where the thermal well is not used, but rather, the sensing probe is used only. The housing configurations may vary as well.

The sensing apparatus, in some embodiments, is used to sense conductivity. In some embodiments, this is in addition to temperature sensing. In those embodiments where both temperature and conductivity sensing is desired, the sensing probe typically includes at least three leads, where two of these leads may be used for temperature sensing and the third used for conductivity sensing.

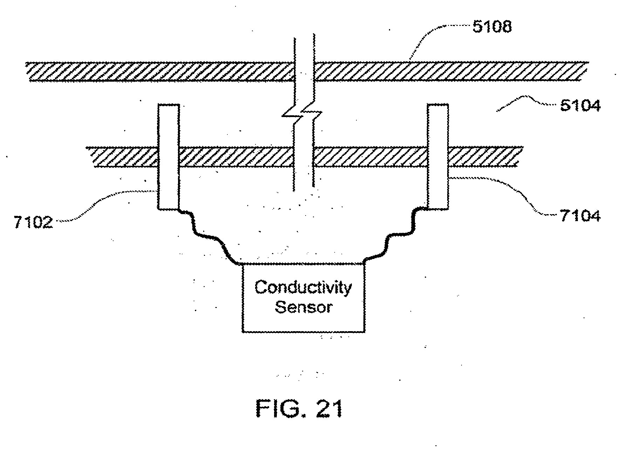

Referring now to FIG. 21, for conductivity sensing, at least two sensors **7102, 7104** are located in an area containing the subject media. In the embodiment shown, the area containing the subject media is a fluid path **5104** inside a fluid line **5108.** The conductivity sensors **7102, 7104** can be one of the various embodiments of sensing probes as described above, or one of the embodiments of the sensor apparatus embodiments (including the thermal well) as described above. However, in other embodiments, only one of the sensors is one of the embodiments of the sensor apparatus or one of the embodiments of the sensing probe, and the second sensor is any electrical sensor known in the art. Thus, in the systems described herein, conductivity and temperature can be sensed through using either one of the sensor apparatus or one of the sensor probes as described herein and a second capacitance sensor, or one of the sensor apparatus or one of the sensor probes as described herein and an electrical sensor.

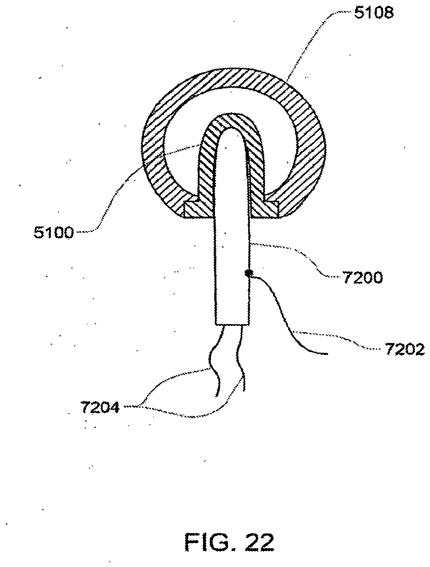

Referring now to FIG. 22, an alternate embodiment of a sensor apparatus including a sensing probe **7200** and a thermal well **5100** is shown in a fluid line **5108.** In this embodiment, the sensing probe **7200** is constructed of a metal housing. The thermal well **5100** is also constructed of metal. The thermal well **5100** and the sensing probe **7200** can be made from the same metal or a different metal. The metal, in the preferred embodiment, is a conductive metal, which may include stainless steel, steel, copper and silver. A lead **7202** is attached to the sensing probe **7200** housing for conductivity sensing. The thermal sensing leads **7204** are attached to a thermal sensor located inside the sensing probe **7200** housing. In this embodiment, therefore, the third lead **7202** (or the lead for conductivity sensing) can be attached anywhere on the sensing probe **7200** because the sensing probe **7200** is constructed of metal. In the previously described embodiments, where the sensing probe housing was constructed of plastic, and the sensing tip constructed of metal, the third lead for conductivity sensing was attached to the sensing tip.

A known volume of subject media may be used to determine conductivity. Thus, two sensors may be used and the volume of fluid between the two sensors can be determined. Conductivity sensing is done with the two electrical contacts (as described above), where one or both can be the sensor apparatus. The volume of subject media between the two contacts is known.

Conductivity sensing is done by determining the conductivity from each of the sensors and then determining the difference. If the difference is above a predetermined threshold, indicating an abnormal difference in conductivity between the first and second sensor (the designations "first" and "second" being arbitrary), then it can be inferred that air may be trapped in the subject media and a bubble detection alarm may be generated to indicate a bubble. Thus, if there is a large decrease in conductivity (and likewise, a large increase in resistance) between the first and second sensor, air could be trapped and bubble presence may be detected.

Leaks in a machine, system, device or container may be determined using the conductivity sensing. Where a sensing apparatus is in a machine, device or system, and that sensing apparatus senses conductivity, in one embodiment, a lead from the sensor apparatus (or electrical contacts) to an analyzer or computer machine may be present.

In some embodiments, the analyzer that analyzes the electrical signals between the contacts is connected to the metal of the machine, device, system or container. If the analyzer senses an electrical signal from the machine, then a fluid leak may be inferred.

### 3.2. SENSOR APPARATUS AND SENSOR APPARATUS SYSTEMS UTILIZED IN CONNECTION WITH A FLUID CASSETTE

The cassette embodiments shown and described in this description include exemplary and some alternate embodiments. However, any variety of cassettes are contemplated that include similar or additional functionality. As well, the cassettes may have varying fluid paths and/or valve placement and may utilize pumping functions, valving functions, and/or other cassette functions. All of these embodiments are within the scope of the invention.

### 3.2.1. FLEXIBLE MEMBRANE FLUID CASSETTE

Fluid cassettes, including flexible membrane fluid cassettes of the types described in U.S. Patent Nos.: 5,350,357 issued September 27, 1994 and entitled Peritoneal Dialysis Systems And Methods Employing A Liquid Distribution And Pumping Cassette That Emulates Gravity Flow; 5,755,683 issued May 26, 1998 and entitled Cassette For Intravenous-Line Flow-Control System; 6,223,130 issued April 24, 2001 entitled Apparatus And Method For Detection Of A Leak In A Membrane Of A Fluid Flow Control System; 6,234,997 issued May 22, 2001 entitled System And Method For Mixing And Delivering Intravenous Drugs; 6,905,479 issued June 14, 2005 entitled Pumping Cartridge Having An Integrated Filter And Method For Filtering A Fluid With The Cartridge; and U.S. Patent Applications: 10,412,658 filed April 10, 2003 entitled System And Method For Delivering A Target Volume Of Fluid; and 10/696,990 filed October 30, 2003 entitled Pump Cassette Bank, all of which are hereby incorporated herein by reference in their entireties, may be used in conjunction with the sensor apparatus and sensor apparatus systems described herein.

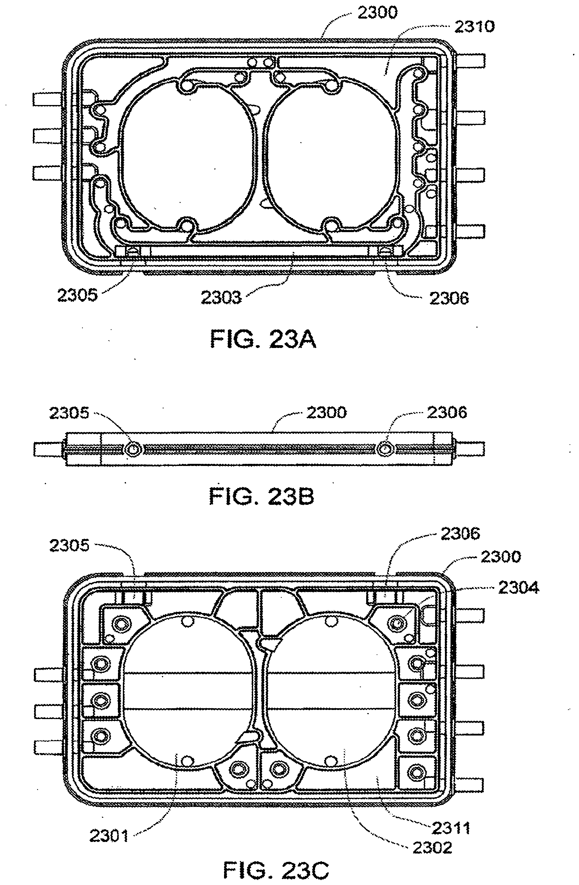

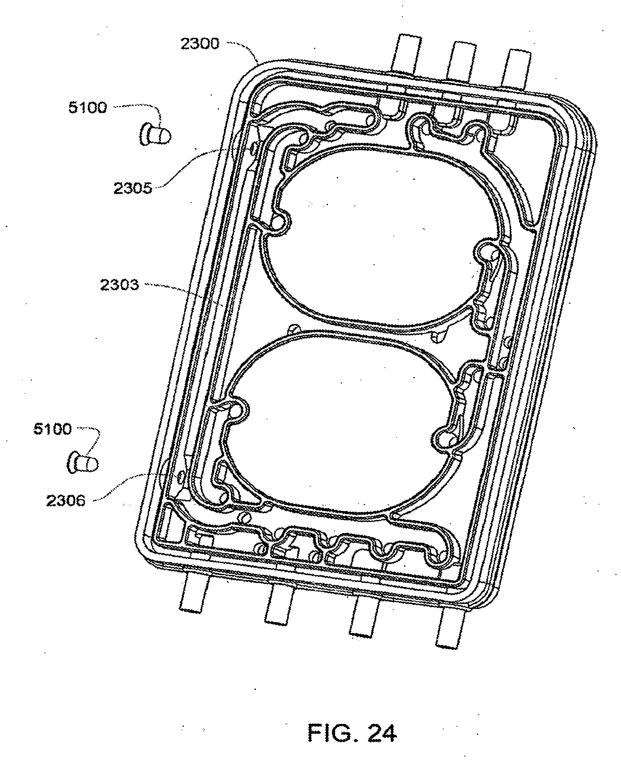

FIGS. 23A-C show an exemplary embodiment of a flexible membrane cassette of a similar type to those generally disclosed in U.S. Patent 5,350,357 and other of the patents and patent applications referenced above. FIGS. 23A-C shows back, side, and front views of exemplary cassette **2300.** As FIGS. 23A-C show, the cassette **2300** includes an injection molded body having back side **2310** shown in FIGS. 23A and front side **2311** shown in FIG. 23C. A flexible diaphragm (one of which is shown as 59 in FIG. 24) overlies the front side and back side of cassette **2300.**

The cassette **2300** is preferably made of a rigid plastic material and the diaphragms are preferably made of flexible sheets of plastic, although many other materials may be utilized.

Exemplary cassette **2300** forms an array of interior cavities in the shapes of wells and channels. In exemplary cassette **2300,** the interior cavities create multiple paths, such as fluid path **2303,** to convey liquid (as FIG. 23A shows). In exemplary cassette **2300,** the interior cavities also create pump chambers, such as pump chambers **2301** and **2302** (as FIG. 23C shows) and multiple valve stations, such as valve station **2304** (as FIG. 23C shows). In the exemplary cassette **2300,** the valve stations, such as valve station **2304,** interconnect the multiple liquid paths, such as fluid path **2303,** with pump chambers **2301** and **2302** and with each other.

In certain embodiments, exemplary cassette **2300** may be utilized in conjunction with a device (not shown) that locally applies positive and negative pressure, including positive and negative fluid pressure of the type described in U.S. Patent 5,350,357 and other of the patents and patent applications referenced above, on the diaphragm regions overlying the valve stations and pump chambers. While many different types of pump chambers and valves may be utilized with cassette of the types described herein (or, in certain embodiments, not included at all), exemplary pump chambers and valve stations of the type shown in FIGS. 23A-C are described in more detail in U.S. Patent 5,350,357, incorporated herein. The presence, number, and arrangement of the pump chambers, liquid paths, and valve stations can vary. Additionally, alternative or additional cassette functionality may be present in a given cassette.

With further reference to FIGS. 23A-C, exemplary cassette **2300** includes sensor ports **2305** and **2306** that extend into fluid path **2303.** Sensor ports **2305** and **2306** may be used to insert a sensing probe, thermal well or other sensing element to allow. Exemplary cassette **2300** shows two sensor ports per cassette, but one port, two ports, or more than two ports may be used depending on the configuration of the cassette and the type of sensor or sensors used.

Again, with reference to FIGS. 23A-C, exemplary cassette **2300** is shown with sensor ports **2305** and **2306** position in the rigid body of cassette **2300.** In the case of a rigid cassette body with two flexible membranes, one on either side of the rigid body, as shown in FIG. 23A-C, in one embodiment sensor ports **2305** and **2306** may be position in the rigid body portion of the cassette (as shown best in FIG. 23B). However, in other embodiments, the sensor port may extend though one or more areas of the flexible diaphragm overlying the cassette.

Referring now to FIG. 24, exemplary cassette **2300** is shown with sensor ports **2305** and **2306** extending into fluid path **2303** such that a component placed in sensor ports **2305** and **2306** would come into direct contact with the subject media contained in or flowing through fluid path **2303.** FIG. 24 additionally shows thermal wells **5100** positioned near sensor ports **2305** and **2306.** In this embodiment, cassette **2300** and thermal wells **5100** are separate parts. In some embodiments, the cassette **2300** and the thermal well **5100** are made from different materials. For these embodiments, the thermal well **5100** can be made from any materials, including but not limited to, plastic, metal, ceramic or a combination thereof. The material may depend in some part on the compatibility with the intended subject media. In other embodiments, thermal well **5100** could be made from the same material as cassette **2300.** In yet further embodiments, thermal well **5100** could be formed as a part of the structure of the rigid body of cassette **2300.**

The length and width of the thermal well **5100** utilized with exemplary cassette **2300** can be any length and width having the desired or tolerable accuracy characteristics and which properly positions any sensor or sensing probe utilized with thermal well **5100** sufficiently in contact with the subject media contained in or flowing through fluid path **2306.** The length of thermal well **5100** may impact the fluid flow of the subject media in fluid path **2303** to a certain extent. It also should be understood that the length of the thermal well **5100** may also impact the turbulence of the fluid flow. Thus, the length and width of the thermal well **5100** may be changed to have greater or lesser impact on the fluid flow and turbulence of the fluid, while mitigating the other variables.

The shape of the thermal well **5100** is also a variable. Any shape desired is contemplated. However, the shape of the thermal well **5100,** as with the other variables, is determined in part based on the intended use of the sensor apparatus. For purposes of description, an exemplary embodiment is described herein. However, the shape in the exemplary embodiment is not meant to be limiting. All of the various embodiments of thermal wells described herein may be used in conjunction with cassettes, such as exemplary cassette **2300.**

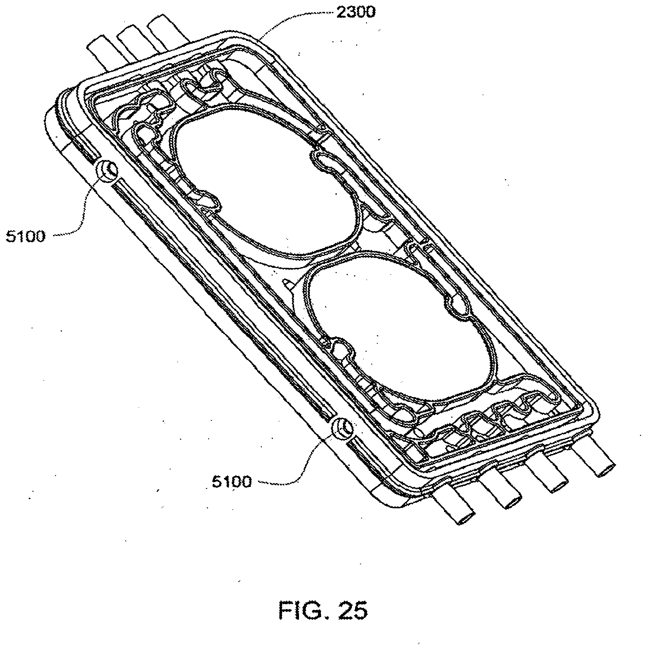

FIG. 25 shows thermal wells **5100** installed in exemplary cassette **2300.** Thermal well **5100** may be installed in exemplary cassette **2300** by use of the ways described herein, including adhesive, welding (ultrasonic and otherwise), o-ring, retaining plate, and otherwise. The thermal well **5100** used in connection with a cassette may be of various shapes and configurations. However, referring now to FIG. 4 for purposes of description, the embodiment of a thermal well **5100** shown may be utilized in conjunction with a cassette. In the exemplary embodiment shown in FIG. 4, the bottom zone **5406** is shaped to aid in press fitting the thermal well into the sensor port **2305** shown in FIGS. 23A-C and 24.

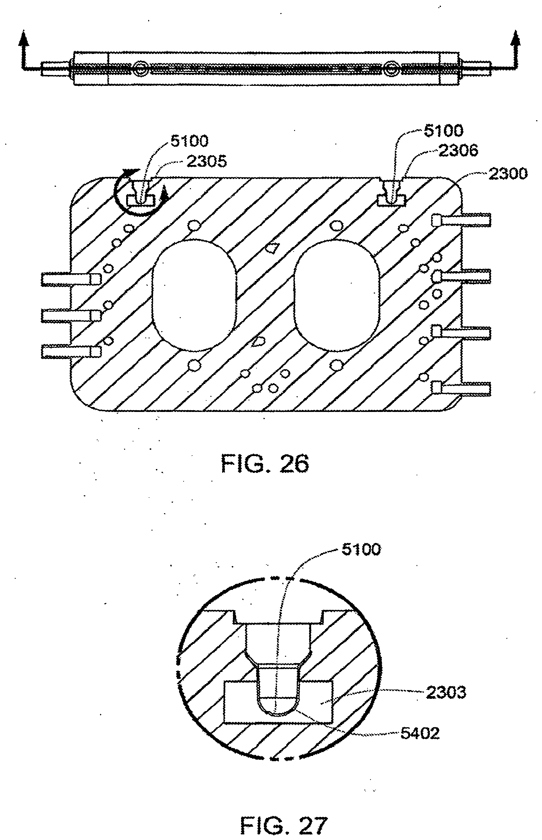

FIG. 26 further shows thermal well **5100** installed in sensor port **2305** and **2306.** As may be best shown by FIG. 27, thermal well **5100** extends into fluid path **2303** so that thermal well **5100** may come into direct contact with any subject media contained in or flowing through exemplary cassette **2300.**

In certain embodiments of sensor apparatus and sensor apparatus systems used in conjunction with a flexible membrane cassette, a sensing probe may be installed directly into sensing ports **2305** and **2306** (sensing ports **2305** and **2306** as shown in FIGS. 23A-C and 24). In further embodiments of sensor apparatus and sensor apparatus systems used in conjunction with a flexible membrane, a sensing probe may be used with a thermal well.

As can be seen in FIG. 27, subject media is in contact with the outside of zone **5402** of the thermal well **5100.** Thermal energy is transferred from the subject media to the thermal well **5100.** As may be seen with reference to FIG. 13A-B, the thermal energy can them be further transferred to the tip **6002** of the sensing probe **6000.** Thermal energy is then conducted to the thermal sensor **6014.** The thermal sensor **6014** communicates via leads **6016** with equipment that can determine the temperature of the subject media based on feedback of the thermal sensor **6014.** In embodiments where conductivity sensing is also desired, lead **6018** communicates with equipment that can determine the conductivity of the subject media. With respect to determining the conductivity of the subject media, in addition to the lead **6018,** a second electrical lead/contact (not shown) would also be used. The second lead could be any probe or apparatus capable of sensing capacitance of the subject media, including, an electrical contact.

Heat transfer from the tip **6002** to the thermal sensor **6014** may be improved by the use of a thermal epoxy or thermal grease **6022.**

Many different embodiments of sensing apparatus may be used in connection with a thermal well installed in a flexible cassette, including embodiments similar to those shown in FIGS. 14A-B, 15, and 16, and described above.

While several geometries have been described, many others could be shown to achieve desired performance characteristics.

In certain embodiments, exemplary cassette **2300** may be utilized in conjunction with a device (not shown) that locally applies positive and negative pressure, including positive and negative fluid pressure of the type described in U.S. Patent 5,350,357 and other of the patents and patent applications referenced above, on the diaphragm regions overlying the valve stations and pump chambers. When cassette **2300** is utilized in conjunction with a pressure applying device (not shown), cassette **2300** may be connected to the device in a number of different ways and in a number of different positions. Preferably, in certain embodiments, cassette **2300** may be loaded in a device in other than a horizontal orientation, such as a vertical or substantially vertical orientation. Placement of the cassette in a vertical or substantially vertical orientation may offer certain advantages depending on the configuration of the cassette such as to avoid air entrapment and to optimize application of positive and negative pressure, including positive and negative fluid pressure of the type described in U.S. Patent 5,350,357 and other of the patents and patent applications referenced above, to the cassette.

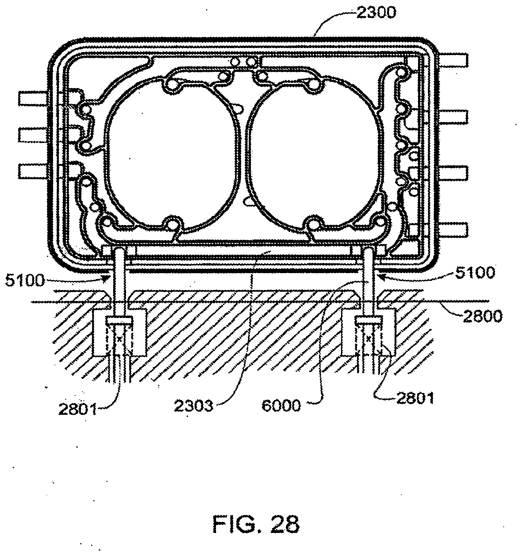

Referring now to FIG. 28, a sensor apparatus system of the type generally shown may be used in connection with exemplary cassette **2300.** In the system, the sensor apparatus is installed in sensor ports **2305** and **2305** (not shown) extending into fluid path **2303.** The sensor apparatus includes the sensing probe **6000** and the thermal well **5100.** In this embodiment, the thermal well **5100** and fluid line **2303** is contained in an exemplary cassette **2300.** In certain embodiments, exemplary cassette **2300** is intended to be **disposable.** Sensing probe **6000** is mounted in a reusable portion. Also in the reusable portion is a spring **2801.** The spring **2801** and sensing probe **6000** are located in a housing **2800.** The housing **2800** can be in any machine, container, device or otherwise. In certain embodiments the reusable portion in contained in or otherwise a part of a pressure applying device (as described above). The spring **2801** can be a conical, a coil spring, wave spring, or urethane spring.

In certain embodiments, the thermal well **5100** and the sensing probe **6000** may include alignment features (of the type shown in FIG. 17, **6702, 6704)** that aid in the thermal well **5100** and sensing probe **6000** being aligned. The correct orientation of the thermal well **5100** and the sensing probe **6000** may aid in the mating of the thermal well **5100** and the sensing probe **6000** to occur. Referring again to FIG. 28, the configuration of the housing **2800** may provide the sensing probe **6000** with space for lateral movement. This allows the sensing probe **6000** to, if necessary; move laterally in order to align with the thermal well **5100** for mating.

In various embodiments, the sensing probe **6000** is configured with respect to the housing **2800** (as shown in FIG. 28) to facilitate engagement between the sensing probe **6000** and the thermal well **5100** and to aid in establishing full contact of the sensing probe **6000** and the thermal well **5100.** Variations of the configurations generally shown in FIGS. 18-20 and described above may be used in conjunction with exemplary cassette **2300.**

In other embodiments, the sensing probe may be aligned and positioned by other housing configurations. Thus, the embodiments of the housing shown herein are only some embodiments of housings in which the sensor apparatus can be used. The sensor apparatus generally depends on being located amply with respect to the subject media. The configurations that accomplish this can vary depending on the subject media and the intended use of the sensing apparatus. Further, in some embodiments where the thermal well is not used, but rather, the sensing probe is used only. The housing configurations may vary as well.

In embodiments in which cassette **2300** is loaded into a device, such as a pressure applying device, in a vertical or substantially vertical orientation, it may be preferable for sensor ports **2305** and **2306** to be positioned in the bottom edge of cassette **2300** (the bottom edge as the cassette is shown in FIG. 23A). Positioning of the sensor ports **2305** and **2306** along the bottom edge of exemplary cassette **2300** (such that sensor ports **2305** and **2306** and installed thermal wells **5100** extend into the bottom fluid line **2303** of the cassette) may facilitate engagement with the sensor apparatus as shown in FIG. 28. In certain of these embodiments, the exemplary cassette **2300** with installed thermal wells **5100** may be placed in position over sensor probes **6000,** and then rotated vertically down and onto the sensor probes **6000.**

The sensing apparatus, in some embodiments, is used to sense conductivity of the subject media within a fluid line within a cassette. In some embodiments, this is in addition to temperature sensing. In those embodiments where both temperature and conductivity sensing is desired, the sensing probe typically includes at least three leads, where two of these leads may be used for temperature sensing and the third used for conductivity sensing.

Referring now to FIG. 21, for conductivity sensing, at least two sensors **7102, 7104** are located in an area containing the subject media. In the embodiment shown, the area containing the subject media is a fluid path **5104** inside a fluid line **5108.** The conductivity sensors **7102, 7104** can be one of the various embodiments of sensing probes as described above, or one of the embodiments of the sensor apparatus embodiments (including the thermal well) as described above.

Referring now to FIG. 28, sensing probes **6000** installed in thermal wells **5100** in sensor ports **2305** and **2306** can be used for sensing the conductivity of the subject media located between sensor ports **2305** and **2306** in fluid line **2303.** However, in other embodiments, only one of the sensors is one of the embodiments of the sensor apparatus or one of the embodiments of the sensing probe, and the second sensor is any electrical sensor known in the art. Thus, in the systems described herein, conductivity and temperature can be sensed through using either one of the sensor apparatus or one of the sensor probes as described herein and a second capacitance sensor, or one of the sensor apparatus or one of the sensor probes as described herein and an electrical sensor.

### 3.2.2. POD PUMP CASSETTE

Cassettes other than the flexible membrane cassette described above may be used in conjunction with the sensor apparatus and sensor apparatus systems described herein. Cassette, such as cassettes of the types described in Patent Application Serial No. 11/787,213 entitled Heat Exchange Systems, Devices and Methods which was filed on April 13, 2007 (E77); Patent Application Serial No. 11/787,213 entitled Fluid Pumping Systems, Devices and Methods which was filed on April 13, 2007 (E78); and Thermal and Patent Application Serial No. 11/787,213 entitled Conductivity Sensing Systems, Devices and Methods which was filed on April 13, 2007 (E79), all of which are hereby incorporated herein by reference in their entireties, may be used in conjunction with the sensor apparatus and sensor apparatus systems described herein. Additionally, cassettes, cassette assemblies, and manifolds of the types described in the following applications may be used in conjunction with the sensor apparatus and sensor apparatus systems described herein: U.S. Patent Application Serial No. 11/871,680, filed October 12, 2007 entitled Pumping Cassette (Attorney Docket No. DEKA-019XX); U.S. Patent Application Serial No.11/871,712, filed October 12, 2007 entitled Pumping Cassette (Attorney Docket No. DEKA-020XX); U.S. Patent Application Serial No. 11/871,787, filed October 12, 2007 and entitled Pumping Cassette (Attorney Docket No. DEKA-021XX); U.S. Patent Application Serial No. 11/871,793, filed October 12, 2007 and entitled Pumping Cassette (Attorney Docket No. DEKA-022XX); and U.S. Patent Application Serial No. 11/871,803, filed October 12, 2007 and entitled Cassette System Integrated Apparatus (Attorney Docket No. DEKA-023XX). Further, a variety of devices, including medical devices, such as the hemodialysis systems and methods of the types described in U.S. Patent Application Serial No. 11/871,680, filed October 12, 2007 entitled Pumping Cassette (Attorney Docket No. DEKA-019XX); as well as U.S. Patent Application entitled Hemodialysis System and Methods (Attorney Docket No. D0570/70019US00) and U.S. Patent Application entitled Cassette System Integrated Apparatus (Attorney Docket No. F62), which are being filed on even date herewith and are hereby incorporated herein by reference in their entirety.

In an exemplary embodiment of other cassettes used in conjunction with the sensor apparatus and sensor apparatus systems described herein, the cassette includes a top plate, a midplate and a bottom plate. In general, the top plate includes pump chambers, and potentially alternative or additional features; the midplate includes complementary fluid lines, metering pumps, valves and potentially alterative or additional features; and the bottom plate includes actuation chambers. In general, membranes are located between the midplate and the bottom plate; however, many alterative embodiments are possible. In the exemplary embodiment, the cassettes are formed by placing the membranes in their correct locations, assembling the plates in order and laser welding the plates. The cassettes may be constructed of a variety of materials. Generally, in the various exemplary embodiment, the materials used are solid and non flexible. In the preferred embodiment, the plates are constructed of polysilicone, but in other embodiments, the cassettes are constructed of any other solid material and in exemplary embodiment, of any thermoplastic.

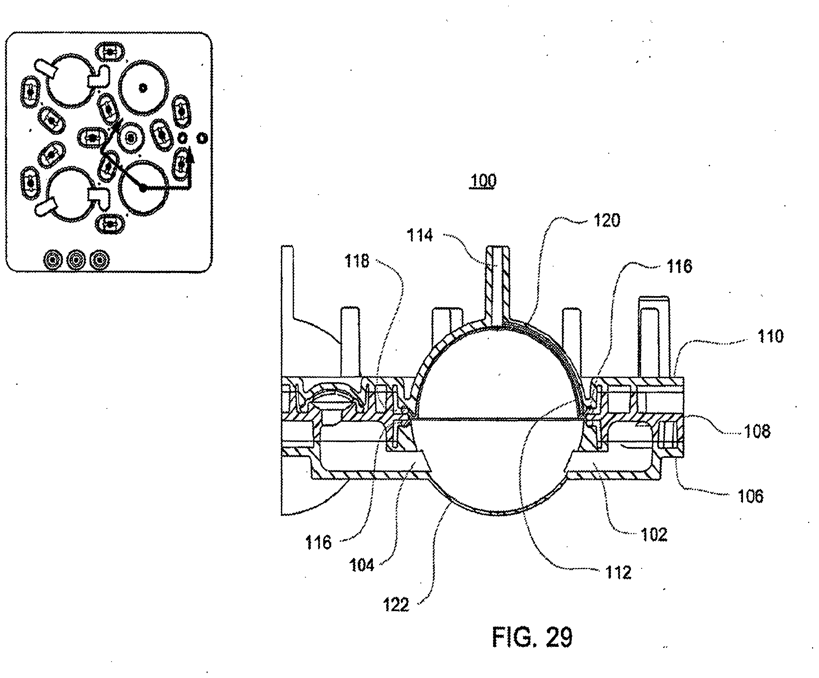

FIG. 29 is a sectional view of an exemplary pump pod 100 that is incorporated into a fluid control or pump cassette, in accordance with an exemplary embodiment of the cassette. In this embodiment, the pump pod is formed from three rigid pieces, namely a "top" plate **106,** a midplate **108,** and a "bottom" plate **110** (it should be noted that the terms "top" and "bottom" are relative and are used here for convenience with reference to the orientation shown in FIG. 29). The top and bottom plates **106** and **110** include generally hemispheroid portions that when assembled together define a hemispheroid chamber, which is a pump pod **100.** A membrane **112** separates the central cavity of the pump pod into two chambers.

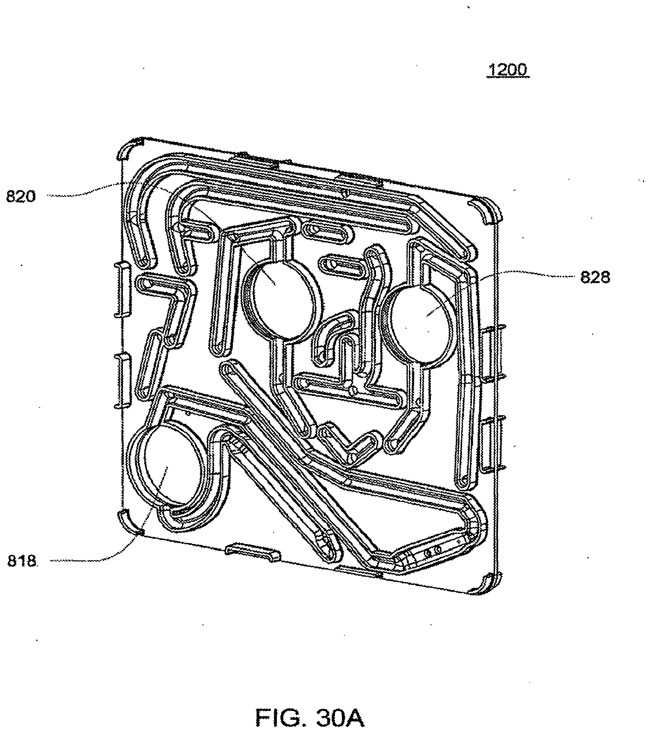

Referring now to FIGS. 30A-B, in the exemplary embodiment of the cassette, sensors are incorporated into the cassette so as to discern various properties of subject media contained in or flowing through the cassette. In various embodiments one sensor may be included to sense temperature and/or other properties of the subject media. In another embodiment, two sensors may be included, to sense temperature and/or conductivity and/or other properties of the subject media. In yet further embodiments, three or more sensors may be included. However, in the exemplary embodiment, 6 sensors (2 sets of 3) are included. The sensors are located in the sensor block **1314, 1316.** In this embodiment, a sensor block **1314, 1316** is included as an area on the cassette for a sensor(s). In the exemplary embodiment, the three sensors of the two sensor blocks **1314, 1316** are housed in respective sensor housings **1308, 1310, 1312** and **1318, 1320, 1322.** In the exemplary embodiment, two of the sensor housings **1308, 1312** and **1318, 1320** accommodate a conductivity sensor and the third sensor housing **1310, 1322** accommodates a temperature sensor. The conductivity sensors and temperature sensor can be any conductivity or temperature sensor in the art. In one embodiment, the conductivity sensor elements (or sensor leads) are graphite posts. In other embodiments, the conductivity sensors elements are posts made from stainless steel, titanium, or any other material of the type typically used for (or capable of being used for) conductivity measurements. In certain embodiments, the conductivity sensors will include an electrical connection that transmits signals from the sensor lead to a sensor mechanism, controller or other device. In various embodiments, the temperature sensor can be any of the temperature sensors commonly used (or capable of being used) to sense temperature.

However, in alternate embodiments, a combination temperature and conductivity sensor is used of the types described above. In such alternate embodiments, thermal wells of the types described above may be installed in the cassette. In such embodiments, thermal well **5100** may be installed in the cassette by use of any of the ways described herein, including adhesive, welding (ultrasonic and otherwise), o-ring, retaining plate, and otherwise.

In alternate embodiments, there are either no sensors in the cassette or only a temperature sensor, only one or more conductivity sensors or one or more of another type of sensor.

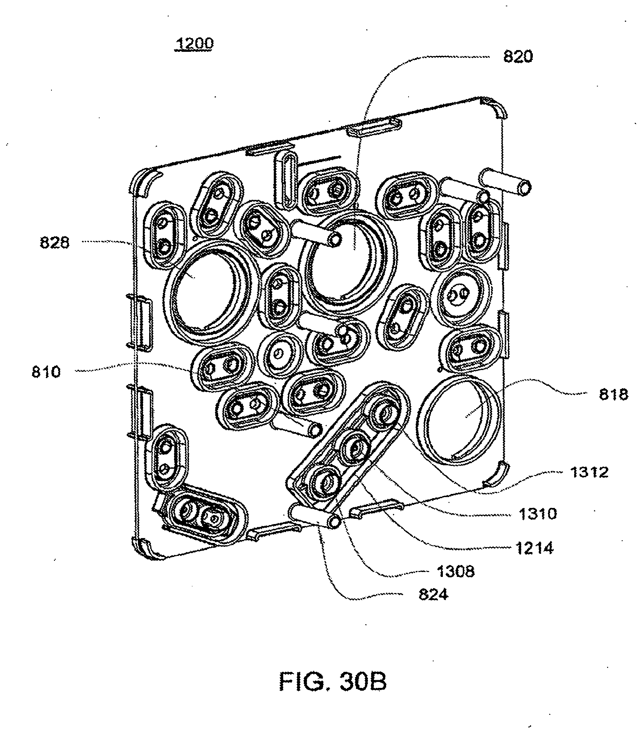

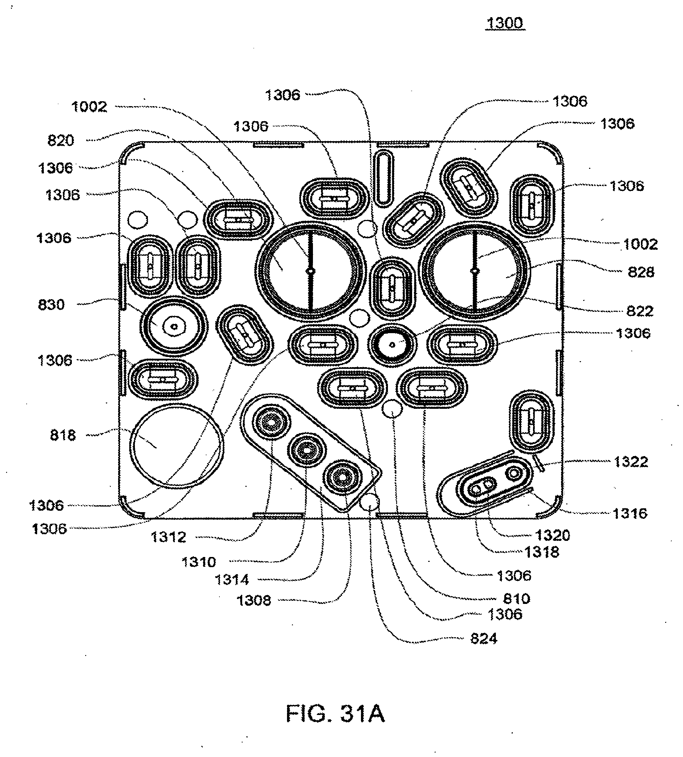

Referring now to FIGS. 31A-13B, the bottom plate **1300** is shown. Referring first to FIGS. 31A, the inner or inside surface of the bottom plate **1300** is shown. The inner or inside surface is the side that contacts the bottom surface of the midplate (not shown). The bottom plate **1300** attaches to the air or actuation lines (not shown). The corresponding entrance holes for the air that actuates the pod pumps **820, 928** and valves (not shown) in the midplate can be seen **1306.** Holes **810, 824** correspond to the first fluid inlet and first fluid outlet shown in FIGS. 30B, **810, 824** respectively. The corresponding halves of the pod pumps **820, 828** and mixing chamber **818** are also shown, as are the raised fluid paths **1002** for the fluid paths. The actuation holes in the pumps are also shown. Unlike the top plate, the bottom plate **1300** corresponding halves of the pod pumps **820, 828** and mixing chamber **818** make apparent the difference between the pod pumps **820, 828** and mixing chamber **818.** The pod pumps **820, 828** include an air/actuation path on the bottom plate **1300,** while the mixing chamber **818** has identical construction to the half in the top plate. The mixing chamber **818** mixes liquid and therefore, does not include a membrane (not shown) nor an air/actuation path. The sensor block **1310, 1316** with the three sensors housings **1308, 1310, 1312** and **1318, 1320, 1322** are also shown.

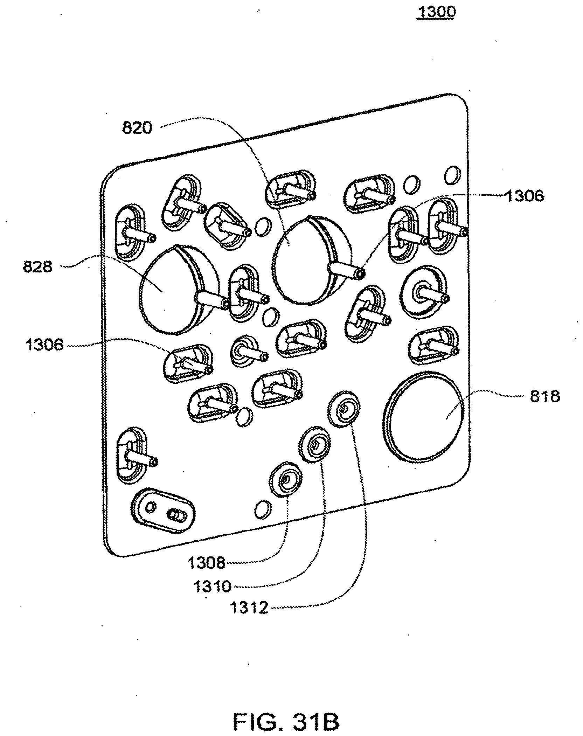

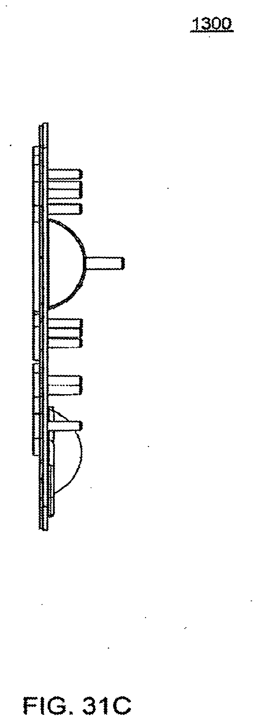

Referring now to FIGS. 31B, the actuation ports **1306** are shown on the outside or outer bottom plate **1300.** An actuation source is connected to these actuation ports **1306.** Again, the mixing chamber **818** does not have an actuation port as it is not actuated by air. Referring to FIG. 31C, a side view of the exemplary embodiment of the bottom plate **1300** is shown.

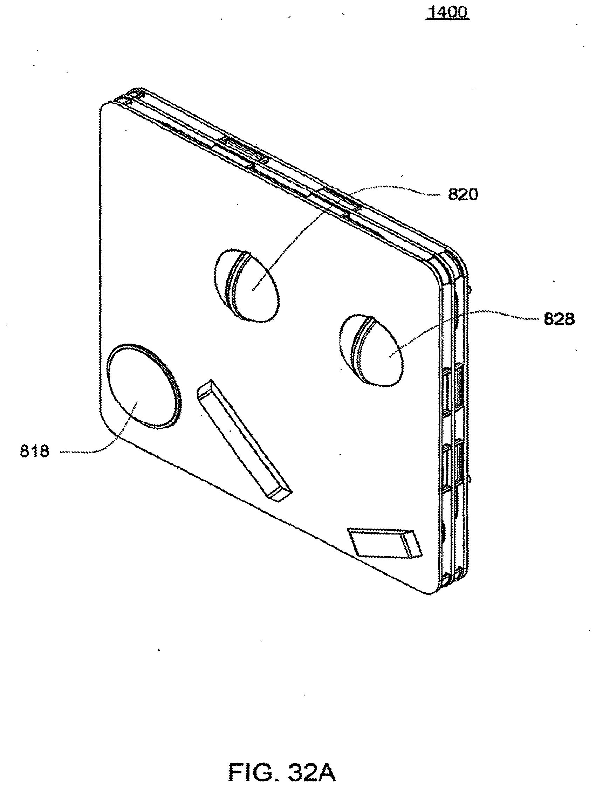

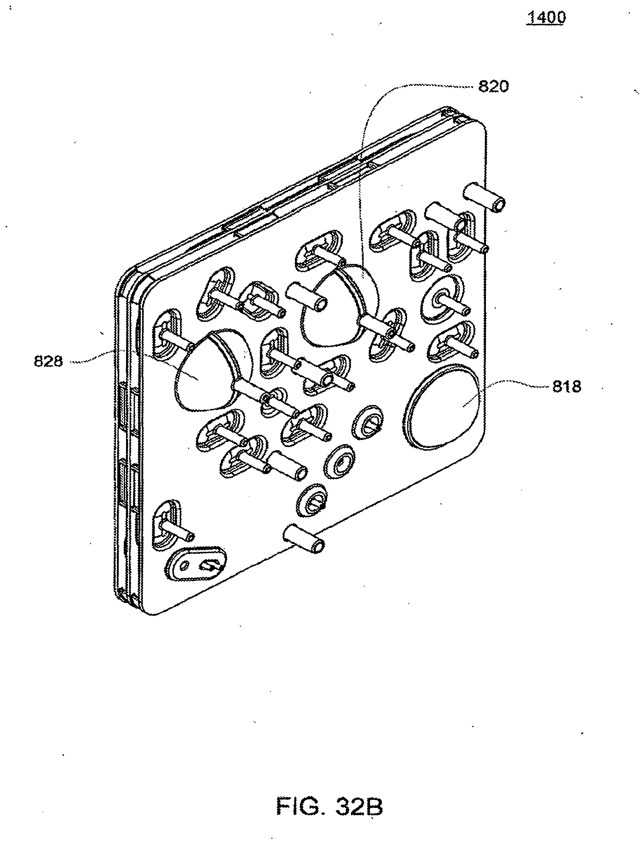

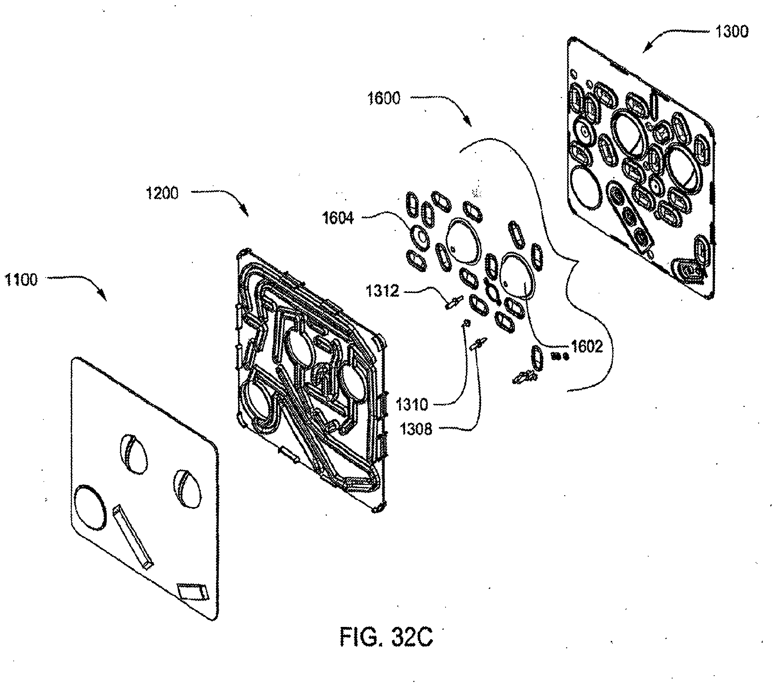

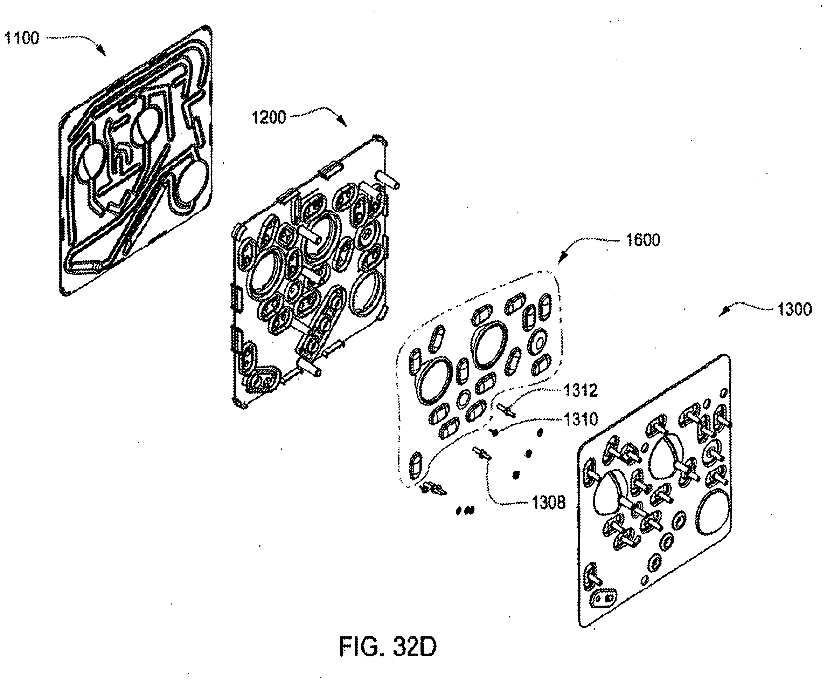

Referring next to FIGS. 32A and 32B, the assembled exemplary embodiment of the cassette **1400** is shown. FIGS. 32C and 32D are exploded view of the exemplary embodiment of the cassette **1400.** One embodiment of the conductivity sensors **1214, 1216** and the temperature sensor **1218,** which make up the sensor cell **1212,** are also shown in FIGS. 32C and 32D. Still referring to FIGS. 32C and 32D, the sensors are housed in sensor blocks (shown as **1314, 1316** in FIGS. 30B and 31A) which include areas on the bottom plate **1300** and the midplate **1200.** O-rings seal the sensor housings from the fluid lines located on the upper side of the midplate **1200** and the inner side of the top plate **1100.** However, in other embodiments, an o-ring is molded into the sensor block or any other method of sealing can be used.

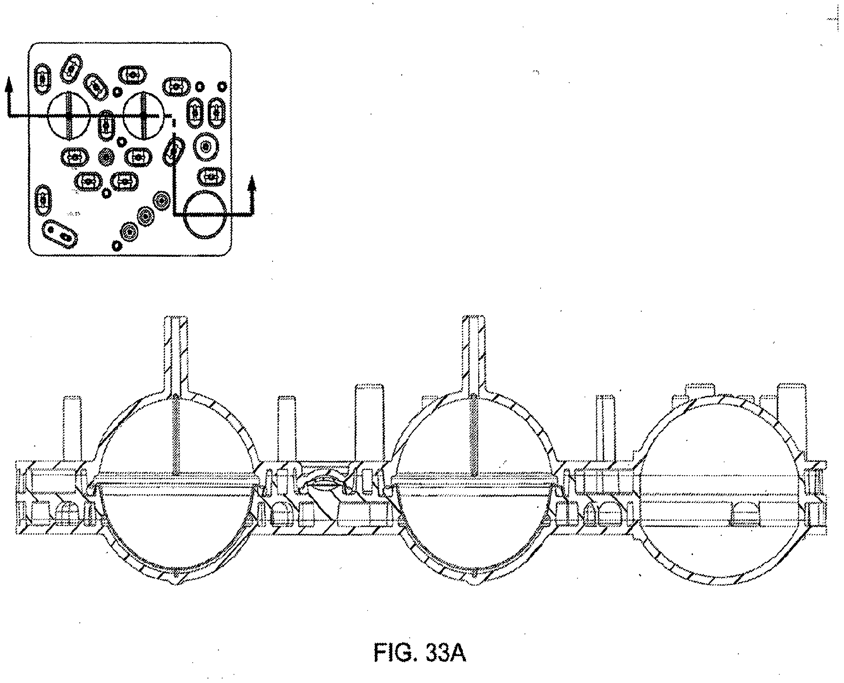

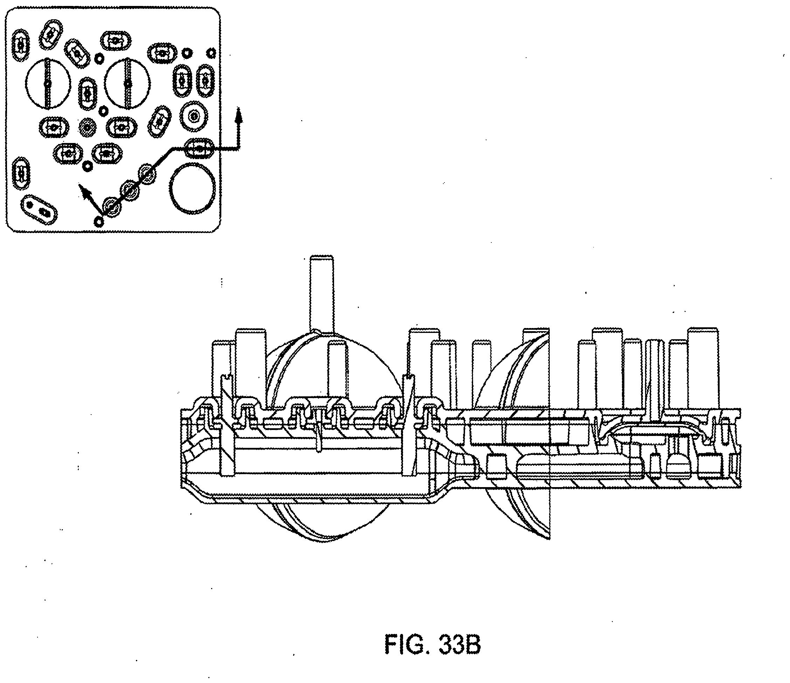

Referring now to FIGS. 33A-33C, various cross sectional views of the assembled cassette are shown. Referring now to FIG. 33B, the two conductivity sensors **1308, 1312** and the temperature sensor **1310** are shown. As can be seen from the cross section, the sensors **1308, 1310, 1312** are in the fluid line **824.** Thus, the sensors **1308, 1310, 1312** are in fluid connection with the fluid line and can determine sensor data of the fluid exiting fluid outlet one **824.** Still referring to FIG. 33B, a valve **826** cross section is shown.

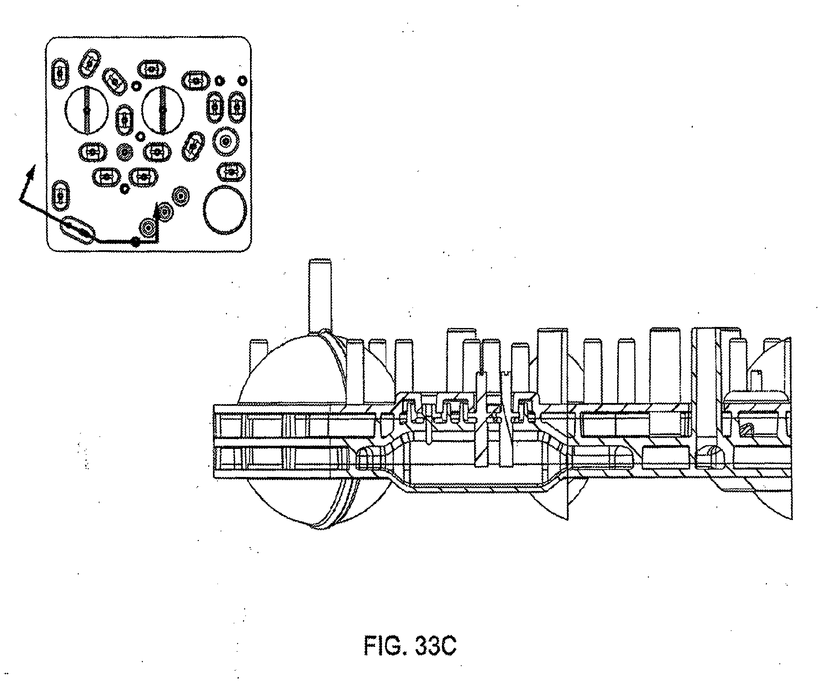

Referring now to FIG. 33C, the two conductivity sensors **1318, 1320** and the temperature sensor **1322** are shown. As can be seen from the cross section, the sensors **1318, 1320, 1322** are in the fluid line **824.** Thus, the sensors **1318, 1320, 1322** are in fluid connection with the fluid line and can determine sensor data of the fluid entering the mixing chamber (not shown in this figure).

Thus, in the exemplary embodiment, the sensors **1318, 1320, 1322** are used to collect data regarding fluid being pumped into the mixing chamber. Referring back to FIG. 30B, sensors **1308, 1310, 1312** are used to collect data regarding fluid being pumped from the mixing chamber and to the fluid outlet. However, in alternate embodiments, no sensors are or only one set, or only one type of sensor (i.e., either temperature or conductivity sensor) is used. Any type of sensor may be used and additionally, any embodiment of a temperature, a conductivity sensor or a combined temperature/conductivity sensor.

### 3.3. SENSOR APPARATUS AND SENSOR APPARATUS SYSTEMS UTILIZED IN CONNECTION WITH A MANIFOLD

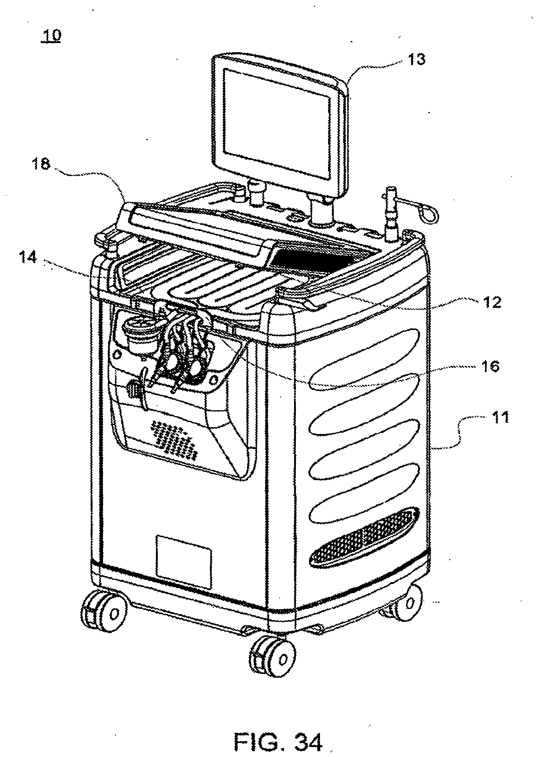

FIG. 34 shows a system **10** in accordance with an exemplary embodiment of the present invention. System **10** includes a base unit **11** and a disposable unit **16** including a manifold. The disposable unit **16** is considered to be "disposable" in that it is generally discarded after a patient treatment, whereas the base unit **11** can be re-used repeatedly by simply installing a new disposable unit **16.**

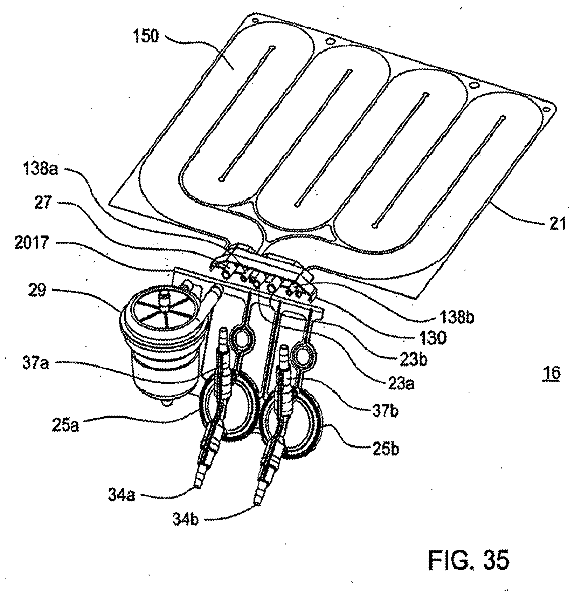

FIG. 35 shows relevant components of a disposable unit **16,** in accordance with an exemplary embodiment of the present invention. The disposable unit **16** includes, among other things, a manifold **130.** The disposable unit **16** preferably also includes a handle (not shown) that is used to mechanically interconnect the above-referenced components into a cohesive unit that can be readily installed into the base unit **11,** which preferably includes a manifold interface (described below) for receiving the manifold **130** and providing pneumatic and other connections. In this embodiment, the manifold **130** is integrated with the heat-exchanger bag **21** and is configured with appropriate tubing connections and supports that are used to interconnect the heat-exchanger bag **21** with the two pump pods **25a** and **25b**. In the embodiment shown in FIG. 35, the manifold **130** includes two flow-path inlets **23a** and **23b** (also referred to as "heat-exchanger bag inlets") in fluid communication with one end of the fluid path **150** and a flow-path outlet **27** (also referred to as a "heat-exchanger bag outlet") in fluid communication with the other end of the fluid path **150.** In alternative embodiments, manifold **130** may be used in connection with disposable unit **16** that does not include a heat-exchanger bag or other components shown in FIG. 35.

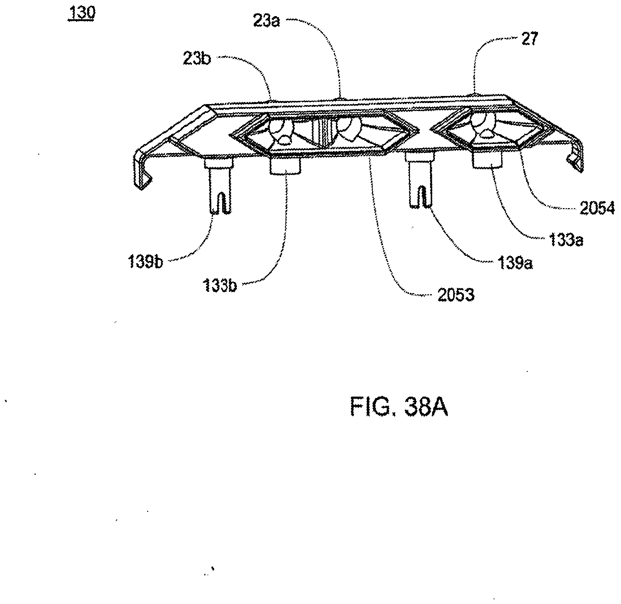

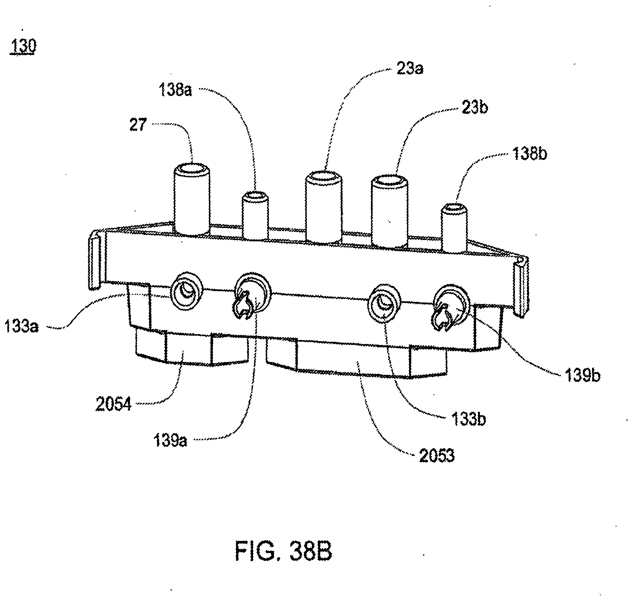

FIGS. 38A and 38B respectively show a perspective back-side view and a perspective bottom view of the manifold **130** from FIG. 35, in accordance with an exemplary embodiment of the present invention. FIG. 38A shows bag inlet and outlet connectors **2053, 2054** for connection at the inlet and outlet openings of the fluid channel **150** of the bag **21.** The bag inlet connector **2053** is in fluid communication with the inlets **23a, 23b,** while the bag outlet connector **2054** is in fluid communication with the outlet **27.** The thermal wells **133a** and **133b** are shown in the outlet fluid path and the inlet fluid path, respectively.

FIG. 13B shows a perspective back-side cross-sectional view of the manifold **130** of FIGS. 35, 38A, and 38B, in accordance with an exemplary embodiment of the present invention. In this embodiment, the manifold **130** includes an inlet thermal well **133a** located in a bag inlet **23a** and an outlet thermal well **133b** located in a bag outlet **27.** The thermal wells **133a, 133b** interface with corresponding probes in a manifold interface of the base unit **11** (discussed below) when the disposable unit **16** is installed in the base unit **11.** FIG. 13C shows a close-up view of an exemplary thermal well, although all of thermal well embodiments described herein may be utilized in connection with a manifold, such as manifold **130.**

The thermal wells **133a, 133b** provide for both thermal and electrical interconnections between the base unit **11** and the disposable unit **16.** Among other things, such thermal and electrical interconnections allow the controller **49** to monitor blood temperature as the blood enters and exits the heat-exchanger bag **21** and also allow the controller **49** to take other measurements (e.g., to detect the presence of blood or air in the heat-exchanger bag **21** and to perform leak detection) as discussed below. In this embodiment, each of the thermal wells **133a, 133b** is coupled so as to have a portion residing directly in the fluid path (i.e., in contact with the blood) so as to permit better transmission of blood temperature from the disposable unit **16** to the base unit **11.** In lieu of, or in addition to, the thermal wells, the disposable unit **16** may include other temperature probes/sensors and interfaces by which the controller **49** can monitor blood temperature as the blood enters and exits the heat-exchanger bag **21.**

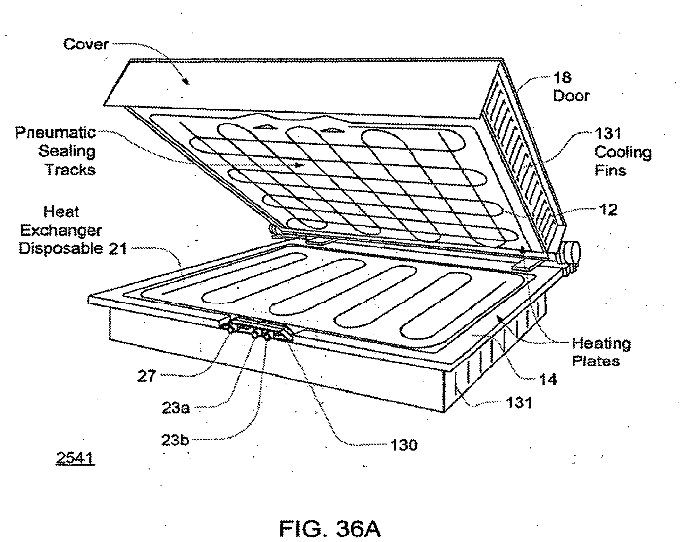

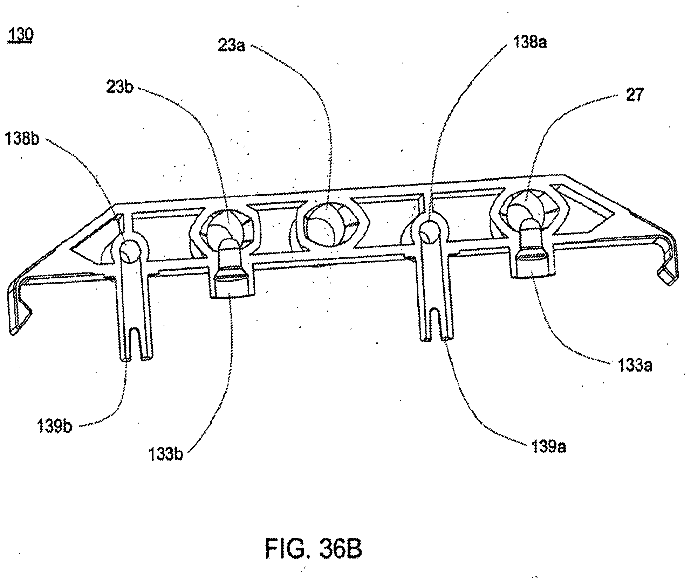

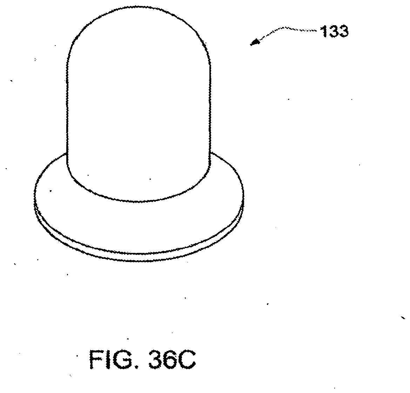

While the exemplary embodiment shown in FIGS. 36B, 38A, and 38B include thermal wells for transmitting thermal information to the base unit **11** and optionally for use in conductivity sensing, it should be noted that other types of sensor components may be additionally or alternatively used. For example, rather than using a thermal well, a sensor component that sends temperature measurements or signals to the base unit **11** may be used. Various types and configurations of sensors are described below. In other embodiments, any of the sensor apparatus and sensor apparatus systems described herein may be used in conjunction with a manifold, such as manifold **130.**

FIG. 26 shows a close-up view of the manifold interface **2500** shown in FIG. 25. The manifold interface **2500** includes, among other things, probes **61, 62** and pneumatic ports **2539a, 2539b**. With reference again to FIG. 13B, it can be seen that the manifold **130** can be installed in the manifold interface **2500** such that the probes **61, 62** interface respectively with the thermal wells **133a, 133b** and the pneumatic ports **2539a, 2539b** interface respectively with the pneumatic interfaces **139a, 139b**. The manifold interface **2500** also includes a data key interface **2540** for interfacing with a corresponding data key in the disposable unit. The data key interface **2540** preferably provides a bi-directional communication interface through which the controller **49** can read information from the disposable unit (e.g., serial/model number, expiration date, and prior usage information) and write information to the disposable unit (e.g., usage information). In an exemplary embodiment, the controller **49** may prevent the start of a treatment if the data key is not present or if the disposable unit is unusable, for example, because it includes an unacceptable serial/model number, is past a pre-configured expiration date, or has already been used. The controller **49** may terminate a treatment if the data key is removed. In lieu of a data key interface **2540,** the base unit **11** or manifold interface **2500** may include other types of interfaces for reading information from the disposable unit and/or writing information to the disposable unit (e.g., RFID, bar code reader, smart key interface).

It should be noted that one or more pumps (e.g., pump pods) may be integral with a manifold such as the manifold **130** and placed in a base unit as a single cartridge. The assembly could include pneumatic connections from the pneumatic ports (which are connected to the base unit) directly to the pump actuation chambers so that no external tubing would be needed to make the pneumatic connections to the pump pods. The assembly could additionally or alternatively include fluidic connections (e.g., from the pump outlets to the interface with the heat-exchanger bag) so that no external tubing would be needed between the pump outlets and the manifold or bag.

### 3.4. SENSOR APPARATUS AND SENSOR APPARATUS SYSTEMS UTILIZED IN CONNECTION WITH A SENSOR MANIFOLD

In various embodiments of the inventions described herein, a sensor apparatus systems may be utilized that comprises a sensor manifold. A sensor manifold may allow subject media to be moved from one environment to another environment that is more conducive to obtaining sensor readings. For example, the cassette manifold may be contained in an area that is not subject to various types of environment conditions, such as temperature and/or humidity, which would not be preferable for sensor apparatus such as a sensing probe. Alternatively, sensing apparatus and sensing apparatus system may be delicate and may be probe to greater malfunctions than other components of a system. Separating the sensor apparatus and the sensor apparatus systems from the remainder of the system by use of a sensor manifold may allow the sensing apparatus and sensing apparatus systems to be repaired or replaced with minimal impact to the remainder of the system. Alternative, the sensor manifold may be replaced either more or less frequently than other components of the system.

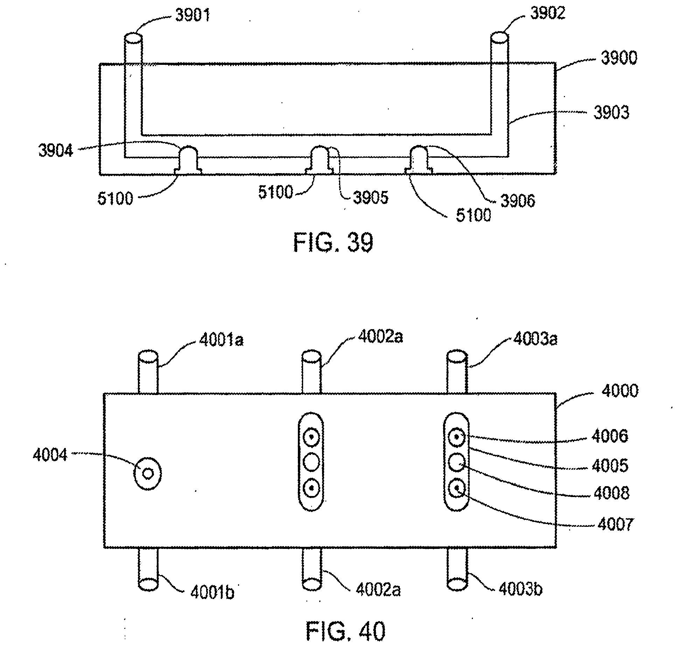

With reference to FIG. 39, an exemplary sensor manifold is shown. A subject media may be contained in or flow through cassette **3900.** In this embodiment, cassette **3900** is comprised of a rigid body overlaid by one or more flexible diaphragms of the types described herein. Pre-molded tube connector **3901** allows subject media to enter sensor cassette **3900** from another source and flow through fluid path **3903.** Subject media exits the cassette through pre-molded tube connector **3902.** While tube connectors **3901** and **3902** are shown as pre-molded tube connectors, other embodiments may use any other fluid transfer devices to allow subject media into fluid path **3903.**

With further reference to FIG. 39, cassette manifold **3900** includes sensor ports **3904, 3905,** and **3906** that extend into fluid path **3903.** Sensor ports **3904, 3905,** and **3906** may be used to insert a sensing probe, thermal well or other sensing element to allow. Exemplary cassette manifold **3900** shows three sensor ports per cassette manifold, but any number of ports may be used depending on the configuration of the cassette manifold and the type of sensor or sensors used.

Again, with reference to FIG. 39, exemplary cassette manifold **3900** is shown with sensor ports **3904, 3905,** and **3906** positioned in the rigid body of cassette manifold **3900.** In the case of a rigid cassette body with two flexible membranes, one on either side of the rigid body, as shown in FIG. 39, in one embodiment sensor ports **3904, 3905,** and **3906** may be position in the rigid body portion of the cassette (as shown in FIG. 39). However, in other embodiments, the sensor port may extend though one or more areas of the flexible diaphragm overlying the cassette manifold.

Referring again to FIG. 39, exemplary cassette manifold **3900** is shown with sensor ports **3904, 3905,** and **3906** extending into fluid path **3903** such that a component placed in sensor ports **3904, 3905,** and **3906** would come into direct contact with the subject media contained in or flowing through fluid path **3903.** FIG. 39 additionally shows thermal wells **5100** installed in sensor ports **3904, 3905,** and **3906.** In certain embodiments, cassette manifold **2300** and thermal wells **5100** are separate parts. In some embodiments, the cassette manifold **3900** and thermal well **5100** are made from different materials. For these embodiments, the thermal well **5100** can be made from any materials, including but not limited to, plastic, metal, ceramic or a combination thereof. The material may depend in some part on the compatibility with the intended subject media. In other embodiments, thermal well **5100** could be made from the same material as cassette manifold **3900.** In yet further embodiments, thermal well **5100** could be formed as a part of the structure of the rigid body of cassette manifold **3900.**

The length and width of the thermal well **5100** utilized with exemplary cassette **2300** can be any length and width having the desired or tolerable accuracy characteristics and which properly positions any sensor or sensing probe utilized with thermal well **5100** sufficiently in contact with the subject media contained in or flowing through fluid path **2306.** The length of thermal well **5100** may impact the fluid flow of the subject media in fluid path **2303** to a certain extent. It also should be understood that the length of the thermal well **5100** may also impact the turbulence of the fluid flow. Thus, the length and width of the thermal well **5100** may be changed to have greater or lesser impact on the fluid flow and turbulence of the fluid, while mitigating the other variables.

The shape of the thermal well **5100** is also a variable. Any shape desired is contemplated. However, the shape of the thermal well **5100,** as with the other variables, is determined in part based on the intended use of the sensor apparatus. For purposes of description, an exemplary embodiment is described herein. However, the shape in the exemplary embodiment is not meant to be limiting. All of the various embodiments of thermal wells described herein may be used in conjunction with cassettes, such as exemplary cassette **2300.**

FIG. 39 shows thermal wells **5100** installed in exemplary cassette manifold **3900.** Thermal well **5100** may be installed in exemplary cassette manifold **3900** by use of the ways described herein, including adhesive, welding (ultrasonic and otherwise), o-ring, retaining plate, and otherwise. The thermal well **5100** used in connection with a cassette may be of various shapes and configurations. However, referring now to FIG. 4 for purposes of description, the embodiment of a thermal well **5100** shown may be utilized in conjunction with a cassette. In the exemplary embodiment shown in FIG. 4, the bottom zone **5406** is shaped to aid in press fitting the thermal well into the sensor port **2304, 3905,** and **3906** shown in FIG. 39. Subject media may come into contact with the outside of zone **5402** of the thermal well **5100** as described above. Thermal energy is transferred from the subject media to the thermal well **5100.** As may be seen with reference to FIG. 13A-B, the thermal energy can them be further transferred to the tip **6002** of the sensing probe **6000.** Thermal energy is then conducted to the thermal sensor **6014.** The thermal sensor **6014** communicates via leads **6016** with equipment that can determine the temperature of the subject media based on feedback of the thermal sensor **6014.** In embodiments where conductivity sensing is also desired, lead **6018** communicates with equipment that can determine the conductivity of the subject media. With respect to determining the conductivity of the subject media, in addition to the lead **6018,** a second electrical lead/contact (not shown) would also be used. The second lead could be any probe or apparatus capable of sensing capacitance of the subject media, including, an electrical contact.

Heat transfer from the tip **6002** to the thermal sensor **6014** may be improved by the use of a thermal epoxy or thermal grease **6022.**

Many different embodiments of sensing apparatus may be used in connection with a thermal well installed in a flexible cassette manifold, including embodiments similar to those shown in FIGS. 14A-B, 15, and 16, and described above.

In certain embodiments of sensor apparatus and sensor apparatus systems used in conjunction with a flexible membrane cassette, a sensing probe may be installed directly into sensing ports **3904, 3905,** and **3906** (shown in FIG. 39). In further embodiments of sensor apparatus and sensor apparatus systems used in conjunction with a flexible membrane, a sensing probe may be used with a thermal well.

In embodiments in which cassette manifold **3900** is used in conjunction with a sensing probe attached to a house, it may be preferable for sensor ports **3904, 3905,** and **3906** to be positioned in the bottom edge of cassette manifold **3900** (the bottom edge as the cassette manifold is shown in FIG. 39). Positioning of the sensor ports **3904, 3905,** and **3906** along the bottom edge of exemplary cassette manifold **3900** (such that sensor ports **2904, 3905,** and **3906** and installed thermal wells **5100** extend into the bottom fluid line **3903** of the cassette) may facilitate engagement with the sensor apparatus as shown in FIG. 28. In certain of these embodiments, the exemplary cassette manifold **3900** with installed thermal wells **5100** may be placed in position over sensor probes **6000,** and then rotated vertically down and onto the sensor probes **6000.**

While several geometries have been described, many others could be shown to achieve desired performance characteristics.

The sensing apparatus, in some embodiments, is used to sense conductivity of the subject media within a fluid line within a cassette. In some embodiments, this is in addition to temperature sensing. In those embodiments where both temperature and conductivity sensing is desired, the sensing probe typically includes at least three leads, where two of these leads may be used for temperature sensing and the third used for conductivity sensing.

Referring now to FIG. 21, for conductivity sensing, at least two sensors **7102, 7104** are located in an area containing the subject media. In the embodiment shown, the area containing the subject media is a fluid path **5104** inside a fluid line **5108.** The conductivity sensors **7102, 7104** can be one of the various embodiments of sensing probes as described above, or one of the embodiments of the sensor apparatus embodiments (including the thermal well) as described above.

Referring now to FIG. 28, sensing probes **6000** installed in thermal wells **5100** in sensor ports **2305** and **2306** can be used for sensing the conductivity of the subject media located between sensor ports **2305** and **2306** in fluid line **2303.** However, in other embodiments, only one of the sensors is one of the embodiments of the sensor apparatus or one of the embodiments of the sensing probe, and the second sensor is any electrical sensor known in the art. Thus, in the systems described herein, conductivity and temperature can be sensed through using either one of the sensor apparatus or one of the sensor probes as described herein and a second capacitance sensor, or one of the sensor apparatus or one of the sensor probes as described herein and an electrical sensor.

For the various embodiments described herein, the cassette may be made of any material, including plastic and metal. The plastic may be flexible plastic, rigid plastic, semi-flexible plastic, semi-rigid plastic, or a combination of any of these. In some of these embodiments the cassette includes one or more thermal wells. In some embodiments one or more sensing probes and/or one or more other devices for transferring information regarding one or more characteristics of such subject media are in direct contact with the subject media. In some embodiments, the cassette is designed to hold fluid having a flow rate or pressure. In other embodiments, one or more compartments of the cassette is designed to hold mostly stagnant media or media held in the conduit even if the media has flow.

In some embodiments, the sensor apparatus may be used based on a need to separate the subject media from the sensing probe. However, in other embodiments, the sensing probe is used for temperature, conductivity, and/or other sensing directly with subject media.

In some embodiments, the thermal well may be part of a disposable portion of a device, machine, system or container. Thus, the thermal well may be in direct contact with subject media and may be the only component that is contaminated by same. In these embodiments, the sensing probe may be part of a machine, device, system or container, and be disposable or non-disposable.

With reference to FIG. 40, another embodiment of an exemplary sensor manifold is shown. A subject media may be contained in or flow through cassette manifold **4000.** Subject media may enter cassette manifold **4000** via pre-molded tube connector **4001a** and exit the cassette manifold via pre-molded tube connector **4001b**. Between tube connector **4001a** and **4001b,** there is a fluid path though the cassette (not shown). Likewise fluid paths (not shown) extend between tube connectors **4002a** and **4002b** and **4003a** and **4003b**.

Referring again to FIG. 40, in this exemplary embodiment of cassettes that may be used in conjunction with the sensor apparatus and sensor apparatus systems described herein, the cassette includes a top plate, a midplate and a bottom plate. Fluid paths, such as the fluid path extending between tube connectors **4001a** and **4001b** extend through the midplate. In the exemplary embodiment, the cassettes are formed by placing the membranes in their correct locations, assembling the plates in order and laser welding the plates. The cassettes may be constructed of a variety of materials. Generally, in the various exemplary embodiment, the materials used are solid and non flexible. In the preferred embodiment, the plates are constructed of polysulfone, but in other embodiments, the cassettes are constructed of any other solid material and in exemplary embodiment, of any thermoplastic.

Referring now to FIG. 40, in an exemplary embodiment of the cassette manifold, sensors are incorporated into the cassette so as to discern various properties of subject media contained in or flowing through the cassette. In various embodiments one sensor may be included to sense temperature and/or other properties of the subject media. In another embodiment, two sensors may be included, to sense temperature and/or conductivity and/or other properties of the subject media. In yet further embodiments, three or more sensors may be included. In some embodiments, such as sensor element **4004,** one sensor element of the type generally described above is included. In other embodiments, the sensors are located in the sensor block **4005.** In this embodiment, a sensor block **4005** is included as an area on the cassette manifold for sensor(s), such as temperature sensors and/or conductivity sensors. The conductivity sensors and temperature sensor can be any conductivity or temperature sensor in the art. In one embodiment, the conductivity sensor elements (or sensor leads) are graphite posts. In other embodiments, the conductivity sensors elements are posts made from stainless steel, titanium, or any other material of the type typically used for (or capable of being used for) conductivity measurements. In certain embodiments, the conductivity sensors will include an electrical connection that transmits signals from the sensor lead to a sensor mechanism, controller or other device. In various embodiments, the temperature sensor can be any of the temperature sensors commonly used (or capable of being used) to sense temperature.

However, in alternate embodiments, a combination temperature and conductivity sensor is used of the types described above. In such alternate embodiments, thermal wells of the types described above may be installed in the cassette. In such embodiments, the thermal well may be installed in the cassette by use of any of the ways described herein, including adhesive, welding (ultrasonic and otherwise), o-ring, retaining plate, and otherwise.

Referring now to FIG. 40, two conductivity sensors **4006** and **4007** and the temperature sensor **4008** are shown. In various embodiments, the sensors **4006, 4007,** and **4008** are in the fluid path (not shown) that extends between tube connectors **4002a** and **4002b** and **4003a** and **4003b.**

### 3.5. FLUID HANDLING SYSTEMS AND METHODS INCLUDING SENSOR APPARATUS AND SENSOR APPARATUS SYSTEMS UTILIZED IN CONNECTION WITH A SENSOR MANIFOLD

In various embodiments of the inventions described herein, systems and methods for fluid handling may be utilized that comprise sensor apparatus systems comprising a sensor manifold. Examples of such embodiments may include systems and methods for the diagnosis, treatment, or amelioration of various medical conditions, including embodiments of systems and methods involving the pumping, metering, measuring, controlling, and/or analysis of various biological fluids and/or therapeutic agents, such as various forms of dialysis, cardio bi-pass, and other types of extracorporeal treatments and therapies. Further examples include fluid treatment and preparation systems, including water treatment systems, water distillation systems, and systems for the preparation of fluids, including fluids utilized diagnosis, treatment, or amelioration of various medical conditions, such as dialysate.

Examples of embodiments of the inventions described herein may include dialysis systems and methods. More specifically, examples of embodiments of the inventions described herein may include hemodialysis systems and methods of the types described in U.S. Patent Application Serial No. 11/871,680, filed October 12, 2007 entitled Pumping Cassette (Attorney Docket No. DEKA-019XX); U.S. Patent Application entitled Hemodialysis System and Methods (Attorney Docket No. D0570/70019US00), filed on even date herewith; and U.S. Patent Application entitled Cassette System Integrated Apparatus (Attorney Docket No. F62), filed on even date herewith.

In such systems and methods, the utilization of one or more sensor manifolds may allow subject media to be moved from one environment to another environment that is more conducive to obtaining sensor readings. For example, the cassette manifold may be contained in an area that is less subject to various types of environment conditions, such as temperature and/or humidity, which would not be preferable for sensor apparatus such as a sensing probe. Alternatively, sensing apparatus and sensing apparatus system may be delicate and may be more prone to malfunctions than other components of a system. Separating the sensor apparatus and the sensor apparatus systems from other components of the system by use of a sensor manifold may allow the sensing apparatus and sensing apparatus systems to be checked, calibrated, repaired or replaced with minimal impact to other components in the system. The ability to check, calibrate, repair or replace the sensor manifold with minimal impact to the remainder of the system may be particularly advantageous when utilized in connection with the integrated cassette systems and methods described in U.S. Patent Application entitled Hemodialysis System and Methods (Attorney Docket No. D0570/70019US00) and U.S. Patent Application entitled Cassette System Integrated Apparatus (Attorney Docket No. F62), which are being filed on even date herewith. Alternatively, the sensor manifold may be replaced either more or less frequently than other components of the system.

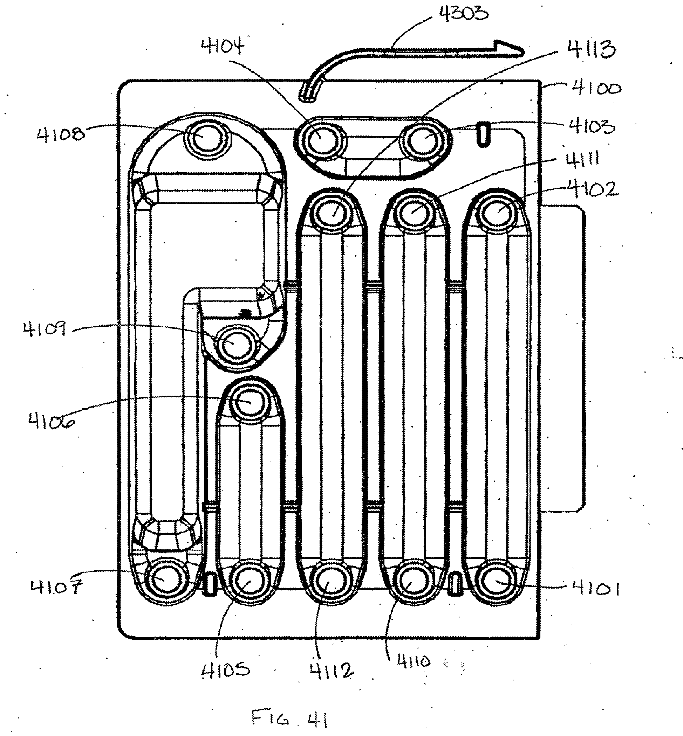

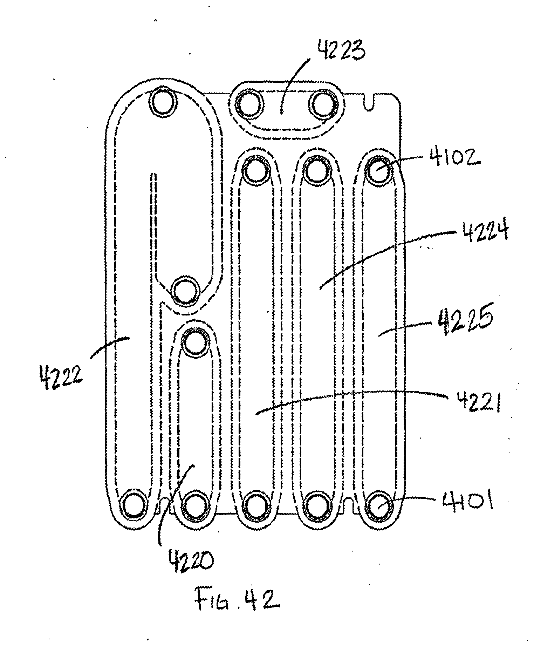

With reference to FIGS. 41 - 46, various other embodiments of an exemplary sensor manifold is shown. One or more subject media, preferably a liquid in these exemplary embodiments, may be contained in or flow through cassette manifold **4100.** For example, one subject media may enter cassette manifold **4100** via pre-molded tube connector **4101** and exit the cassette manifold via pre-molded tube connector **4102.** Between tube connector **4101** and **4102,** there is a fluid path though the cassette (best shown as fluid path **4225** in FIG. 42). Likewise fluid paths (shown as fluid paths **4223, 4220, 4222, 4224,** and **4221** respectively in FIG. 42) extend between sets of tube connectors **4103** and **4104; 4105** and **4106; 4107, 4108,** and **4109; 4110** and **4111;** and **4112** and **4113.** In certain embodiments, each fluid path may contain subject media of different composition or characteristics. In other embodiments, one or more fluid paths may contain the same or similar subject media. In certain embodiments, the same subject media may be flowed through more than one flow path at the same time to check and/or calibrate the sensor apparatus systems associated with such fluid paths.

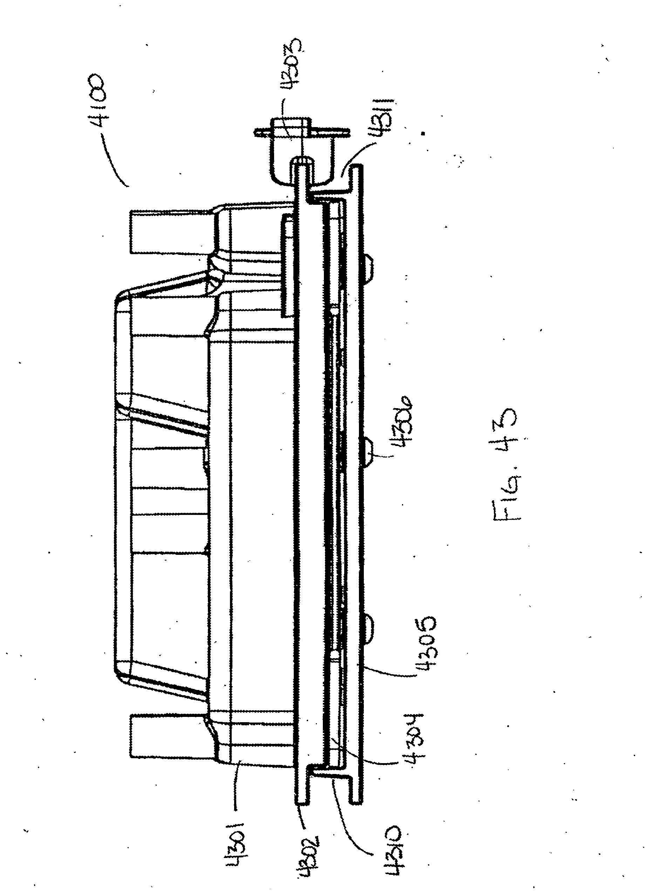

Referring now to FIG. 43, in these exemplary embodiments of sensor manifold **4100** that may be used in conjunction with the sensor apparatus and sensor apparatus systems described herein, the cassette includes a top plate **4302** and a base **4301.** Fluid paths, such as the fluid path **4225** (as shown in FIG. 42) extending between tube connectors **4101** and **4102** extend between the base and top plate. The cassettes may be constructed of a variety of materials. Generally, in the various exemplary embodiment, the materials used are solid and non flexible. In the preferred embodiment, the plates are constructed of polysulfone, but in other embodiments, the cassettes are constructed of any other solid material and in exemplary embodiments, of any thermoplastic. Preferred embodiments of sensor manifold **4100** may be fabricated utilizing the systems and methods described in U.S. Patent Application entitled Cassette System Integrated Apparatus (Attorney Docket No. F62), which is being filed on even date herewith.

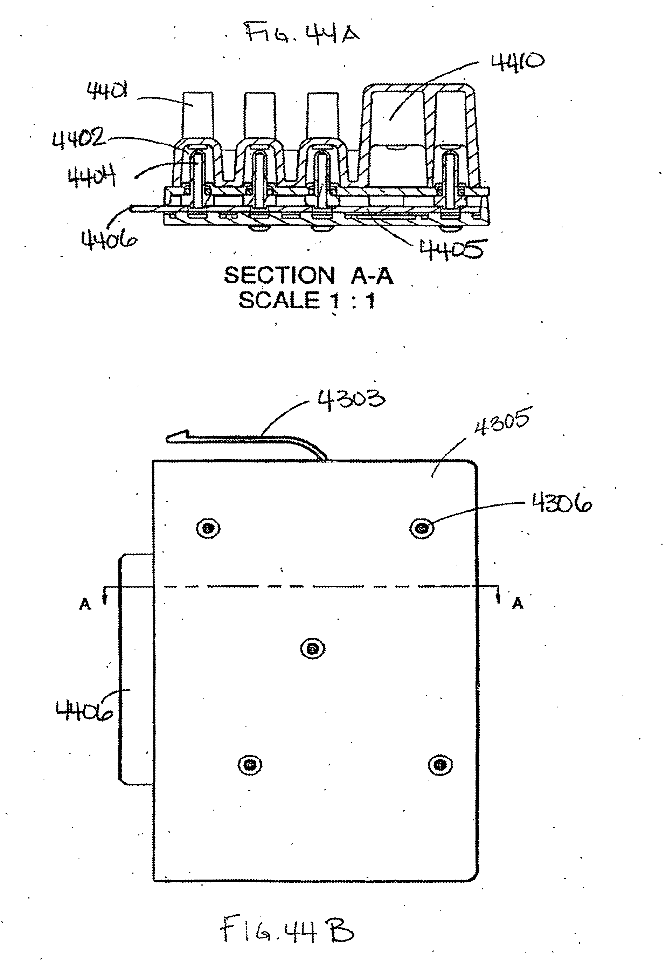

Referring again to FIG. 43, in these exemplary embodiments of sensor manifolds that may be used in conjunction with the sensor apparatus and sensor apparatus systems described herein, the sensor manifold **4100** may also include printed circuit board (PCB) **4304** and a PCB cover **4305.** Various embodiments may also include connector **4303** (also shown in FIGS. 41 and 44B) which may be utilized to mechanically connect the cassette manifold **4100** to the system, such as a hemodialysis system. Cassette manifold **4100** may also utilize various means to hold the layers of sensor manifold **4100** together as a unit. In various embodiments, as shown in FIG. 43, connectors **4306** (also shown in FIG 44B), which in one embodiment is a screw, but in other embodiments may be any means for connection, are utilized, but any means known to one of skill in the art, such as other types of screws, welds, clips, clamps, and other types of chemical and mechanical bonds may be utilized.

Referring now to FIG. 44A, in exemplary embodiments of the sensor manifold **4100,** tube connectors, such as tube connector **4401,** is utilized to bring subject media into or remove subject media from fluid path **4402.** Sensing probes, such as sensing probe **4404** extending into fluid path **4402,** are incorporated into sensor manifold **4100** so as to determine various properties of the subject media contained in or flowing through the particular fluid path in the sensor manifold. In various embodiments one sensing probe may be utilized to sense temperature and/or other properties of the subject media. In another embodiment, two sensing probes may be utilized to sense temperature and/or conductivity and/or other properties of the subject media. In yet further embodiments, three or more sensing probes may be included. In some embodiments, one or more combination temperature and conductivity sensing probes of the types generally described herein may be utilized. In other embodiments, the conductivity sensors and temperature sensor can be any conductivity or temperature sensor in the art. In one embodiment, the conductivity sensor elements (or sensor leads) are graphite posts. In other embodiments, the conductivity sensors elements are posts made from stainless steel, titanium, or any other material of the type typically used for (or capable of being used for) conductivity measurements. In certain embodiments, the conductivity sensors will include an electrical connection that transmits signals from the sensor lead to a sensor mechanism, controller or other device. In various embodiments, the temperature sensor can be any of the temperature sensors commonly used (or capable of being used) to sense temperature.

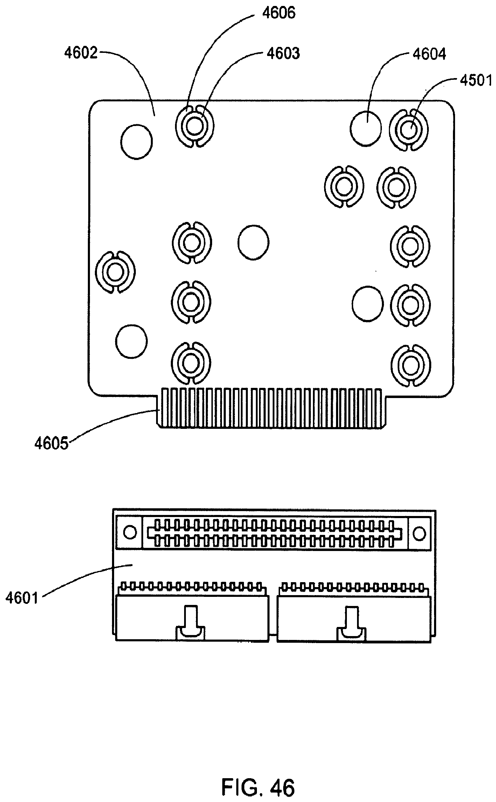

Referring again to FIG. 44A, sensing probe **4404** is electrically connected to PCB **4405.** In certain embodiments, an electrically conductive epoxy is utilized between sensor element **4404** and PCB **4405** to ensure appropriate electrical connection, although other means known to those of skill in the art may be used to obtain an appropriate electrical connection between sensor element **4404** and PCB **4405.** PCB **4405** is shown with edge connector **4406.** In various embodiments, edge connector **4406** may be used to transmit sensor information from cassette manifold **4100** to the main system, such as embodiments of the hemodialysis system described in U.S. Patent Application entitled Hemodialysis System and Methods (Attorney Docket No. D0570/70019US00). Edge connector **4406** may be connected to a media edge connector (such as media edge connector **4601** shown in FIG. 46). In various embodiments, media edge connector **4601** may be installed in a hemodialysis machine (not shown). In such embodiments, guide tracks **4310** and **4311** (as shown in FIG. 43) may be utilized to assist in the connection of edge connector **4406** and media edge connector **4601.** Various embodiments may also include connector **4303** (as shown in FIGS. 41, 43 and 44B) which may be utilized to mechanically connect the cassette manifold **4100** to the system, such as a hemodialysis system.

Referring again to FIG. 44A, air trap 4410 is shown. In certain embodiments, an air trap, such as air trap **4410,** may be utilized to trap and purge air in the system. As may be best shown in FIG. 42, subject media may flow through fluid path **4222** between tube connectors **4107** and **4109** in sensor manifold **4100.** As the flow of the subject media is slowed around the turn in fluid path **4222** (near tube connector **4108**), air may be removed from the subject media through connector **4108.**

Referring now to FIG. 44B, PCB cover **4305** is shown. PCB cover **4305** may be connected to sensor manifold **4100** by connectors **4306.** Edge connector **4406** is also shown.

In accordance with certain embodiments, sensor manifold **4100** is passive with respect to control of the fluid flow. In such embodiments, sensor manifold **4100** does not contain valves or pumping mechanisms to control the flow of the subject media. In such embodiments, the flow of the subject media may be controlled by fluid control apparatus external to sensor manifold **4100.** In other embodiments, the sensor manifold may include one or more mechanical valves, pneumatic valves or other type of valve generally used by those of skill in the art. In such embodiments, the sensor manifold may include one or more pumping mechanisms, including pneumatic pumping mechanisms, mechanical pumping mechanisms, or other type of pumping mechanisms generally used by those of skill in the art. Examples of such valves and pumping mechanisms may include the valves and pumping mechanisms described in U.S. Patent Application Serial No.11/871,680, filed October 12, 2007 entitled Pumping Cassette (Attorney Docket No. DEKA-019XX); U.S. Patent Application entitled Hemodialysis System and Methods (Attorney Docket No. D0570/70019US00), filed on even date herewith; and U.S. Patent Application entitled Cassette System Integrated Apparatus (Attorney Docket No. F62), filed on even date herewith.

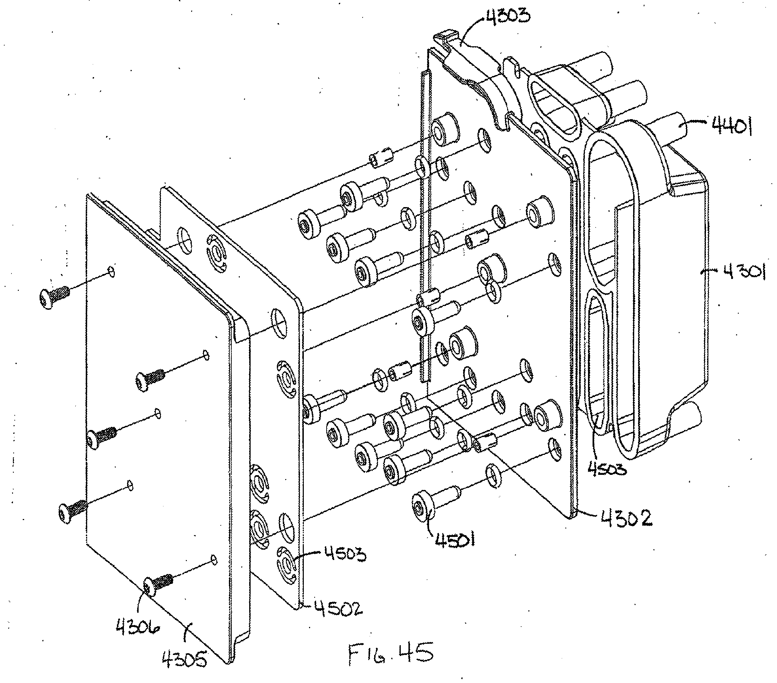

Referring now to FIG. 45, tube connector **4401** is shown in base **4301.** Top plate **4302** is shown, along with connector **4303.** Sensing probes, such as sensing probe **4501,** extend through top plate **4302** into fluid path **4503.** Sensing probe **4501** may be various types of sensors, including the embodiments of sensing probes generally shown in FIGS. 8 and 9 herein.

The sensing probes, such as sensing probe **4501,** may be all the same, may be individually selected from various sensors based on the type of function to be performed, or the same probe may be individually modified based on the type of function to be performed. Similarly, the configuration of the fluid paths, such as the length of the fluid path and the shape of the fluid path, may be selected based on the function to be performed. By way of example, to detect the temperature of the subject media in a fluid path, a temperature sensor, such as a thermistor, may be used. Again, by way of example, to measure the conductivity of the subject media, one sensing probe configured to measure temperature and conductivity, such as sensing probes of the type generally shown in FIGS. 8 and 9, and one sensing probe configured only to measure conductivity may be utilized. In other embodiments, two or more sensing probes configured to measure both temperature and conductivity, such as sensing probes of the type generally shown in FIGS. 8 and 9, may be utilized. In various embodiments of such configurations, by way of example, the second temperature sensor may be present but not utilized in normal operation, or the second temperature may be utilized for redundant temperature measurements, or the or the second temperature may be utilized for redundant temperature measurements.

Referring again to FIG. 45, PCB **4502** is shown with electrical connection **4503.** As further shown in FIG. 46, PCB **4602** is shown with electrical connection **4603** for connection to a sensing probe (shown as **4501** in FIG. 45). PCB **4602** also contains opening **4604** for attachment to top plate (shown as **4305** in FIG. 45). In certain embodiments, electrical connection **4603** is mounted onto, or manufactured with, PCB **4602** with air gap **4606.** In such embodiments, air gap **4606** may be utilized to provide protection to the electrical connection between sensing probe **4501** and PCB **4602** by allowing shrinking and expansion of the various components of sensor manifold **4100** with lesser impact to PCB **4602.**

Referring again to FIG. 46, PCB **4602** is also shown with edge connector **4605.** As described herein, edge connector **4605** may interface with edge connector receiver **4601,** which may be connected to the system, such as the hemodialysis system, to which sensor manifold **4100** interfaces.

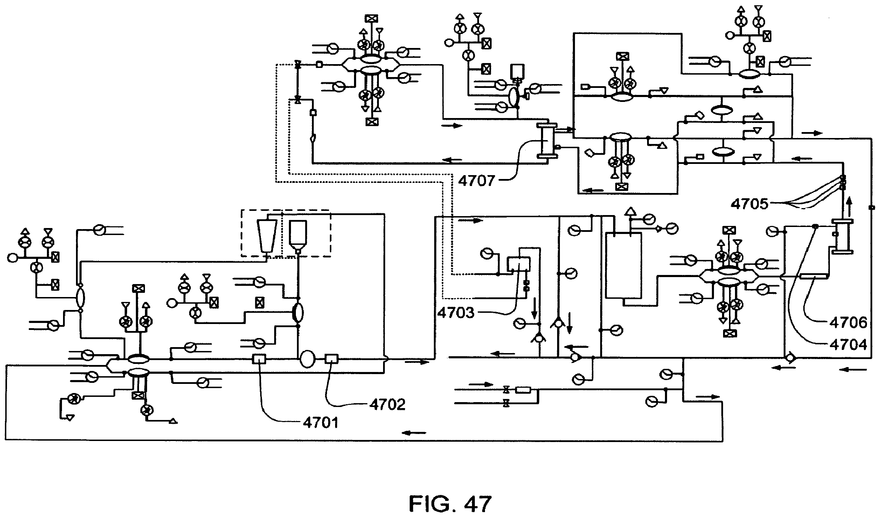

Various embodiments of exemplary sensor manifold **4100** shown in FIG. 41-46 may be utilized in conjunction with hemodialysis systems and methods desscribed in U.S. Patent Application Serial No. 1 1/871,680, filed October 12, 2007 entitled Pumping Cassette (Attorney Docket No. DEKA-019XX); U.S. Patent Application entitled Hemodialysis System and Methods (Attorney Docket No. D0570/70019US00), filed on even date herewith; and U.S. Patent Application entitled Cassette System Integrated Apparatus (Attorney Docket No. F62), filed on even date herewith. In certain embodiments, sensor manifold **4100** contains all of the temperature and conductivity sensors shown in FIG. 47. FIG. 47 depicts a fluid schematic in accordance with one embodiment of the inventions described in the patent applications reference above.

By way of example, in various embodiments, the temperature and conductivity of the subject media at position **4701** as shown in FIG. 47 may be determined utilizing sensor manifold **4100.** In such embodiments, subject media flows into tube connector **4105** (as shown in FIG. 41) through fluid path **4220** (as shown in FIG. 42) and exits at tube connector **4106** (as shown in FIG. 41). The conductivity of the subject media is measured by two sensing probes (not shown) extending into fluid path **4220,** at least one of which has been configured to include a temperature sensing element, such as a thermistor. The conductivity measurement or the temperature measurement of the subject media may be utilized to determine and/or correlate a variety of information of utility to the hemodialysis system. For example, in various embodiments at position **4701** in FIG. 47, the subject media may be comprised of water to which a bicarbinated based solution has been added. Conductivity of the subject media at position **4701** may be utilized to determine if the appropriate amount of the bicarbonate based solution has been added prior to position **4701.** In certain embodiments, if the conductivity measurement deviates from a predetermined range or deviates from a predetermined measurement by more than a predetermined amount, then the subject media may not contain the appropriate concentration of the bicarbonate based solution. In such instances, in certain embodiments, the hemodialysis system may be alerted.

Again, by way of example, in various embodiments, the conductivity of the subject media at position **4702** as shown in FIG. 47 may be determined utilizing sensor manifold **4100.** In such embodiments, subject media flows into tube connector **4112** (as shown in FIG. 41) through fluid path **4221** (as shown in FIG. 42) and exits at tube connector **4113** (as shown in FIG. 41). The conductivity of the subject media is measured by two sensing probes (not shown) extending into fluid path **4221,** at least one of which has been configured to include a temperature sensing element, such as a thermistor. The conductivity measurement or the temperature measurement of the subject media may be utilized to determine and/or correlate a variety of information of utility to the hemodialysis system. For example, in various embodiments at position **4702** in FIG. 47, the subject media may be comprised of water to which a bicarbinated based solution and then an acid based solution has been added. Conductivity of the subject media at position **4702** may be utilized to determine if the appropriate amount of the acid based solution (and the bicarbonate based solution in a previous step) has been added prior to position **4702.** In certain embodiments, if the conductivity measurement deviates from a predetermined range or deviates from a predetermined measurement by more than a predetermined amount, then the subject media may not contain the appropriate concentration of the acid based solution and the bicarbonate based solution. In such instances, in certain embodiments, the hemodialysis system may be alerted.

By way of further example, in various embodiments, the temperature and conductivity of the subject media at position **4703** as shown in FIG. 47 may be determined utilizing sensor manifold **4100.** In such embodiments, subject media may flow into or out of tube connector **4107** (as shown in FIG. 41) through fluid path **4222** (as shown in FIG. 42) and may flow into or out of tube connector **4109** (as shown in FIG. 41). As described herein, air may be removed from the subject media as it moves past the turn in fluid path **4222.** In such instances, a portion of the subject media may be removed through tube connector **4108** to the drain, bringing with it air from the air trap. The conductivity of the subject media is measured by two sensing probes (not shown) extending into fluid path **4222,** at least one of which has been configured to include a temperature sensing element, such as a thermistor. The conductivity measurement or the temperature measurement of the subject media may be utilized to determine and/or correlate a variety of information of utility to the hemodialysis system. For example, in various embodiments, the conductivity measurement at position **4703** in FIG. 47 may be utilized to correlate to the clearance of the dialyzer. In such instances, in certain embodiments, this information may then be sent to the hemodialysis system.

Again, by way of further example, in various embodiments, the temperature of the subject media at position **4704** as shown in FIG. 47 may be determined utilizing sensor manifold **4100.** In such embodiments, subject media flows into tube connector **4103** (as shown in FIG. 41) through fluid path **4223** (as shown in FIG. 42) and exits at tube connector **4104** (as shown in FIG. 41). The temperature of the subject media is measured by one or more sensing probes (not shown) extending into fluid path **4223.** The temperature measurement of the subject media at position **4704** may be utilized to determine and/or correlate a variety of information of utility to the hemodialysis system. For example, in various embodiments at position **4704** in FIG. 47, the temperature of the subject media is determined down stream of a heating apparatus **4706.** If the temperature deviates from a predetermined range or deviates from a predetermined measurement by more than a predetermined amount, then the hemodialysis system may be alerted. For example in certain embodiments, the subject media may be re-circulated through the heating apparatus **4706** until the temperature of the subject media is within a predetermined range.

Again, by way of further example, in various embodiments, the temerparure and conductivity of the subject media at position **4705** as shown in FIG. 47 may be determined utilizing sensor manifold **4100.** In such embodiments, subject media flows into tube connector **4110** (as shown in FIG. 41) through fluid path **4224** (as shown in FIG. 42) and exits at tube connector **4111** (as shown in FIG. 41). The conductivity of the subject media is measured by two sensing probes (not shown) extending into fluid path **4224,** at least one of which has been configured to include a temperature sensing element, such as a thermistor. The conductivity measurement or the temperature measurement of the subject media may be utilized to determine and/or correlate a variety of information of utility to the hemodialysis system. For example, the temperature and conductivity measurement at position **4705** may be used as a further safety check to determine if the temperature, conductivity, and, by correlation, the composition of, the subject media is within acceptable ranges prior to the subject media reaching the dialyzer **4707** and, thus, the patient. In certain embodiments, if the temperature and/or conductivity measurement deviates from a predetermined range or deviates from a predetermined measurement by more than a predetermined amount, then the hemodialysis system may be alerted.

For the various embodiments described herein, the cassette may be made of any material, including plastic and metal. The plastic may be flexible plastic, rigid plastic, semi-flexible plastic, semi-rigid plastic, or a combination of any of these. In some of these embodiments the cassette includes one or more thermal wells. In some embodiments one or more sensing probes and/or one or more other devices for transferring information regarding one or more characteristics of such subject media are in direct contact with the subject media. In some embodiments, the cassette is designed to hold fluid having a flow rate or pressure. In other embodiments, one or more compartments of the cassette is designed to hold mostly stagnant media or media held in the conduit even if the media has flow.

In some embodiments, the sensor apparatus may be used based on a need to separate the subject media from the sensing probe. However, in other embodiments, the sensing probe is used for temperature, conductivity, and/or other sensing directly with subject media.

Although the above discussion discloses various exemplary embodiments of the invention, it should be apparent that those skilled in the art can make various modifications that will achieve some of the advantages of the invention without departing from the true scope of the invention. While the principles of the invention have been described herein, it is to be understood by those skilled in the art that this description is made only by way of example and not as a limitation as to the scope of the invention. Other embodiments are contemplated within the scope of the present invention in addition to the exemplary embodiments shown and described herein. Modifications and substitutions by one of ordinary skill in the art are considered to be within the scope of the present invention.

Further novel and inventive feature combinations are set out in the following numbered statements.
1. A cassette comprising a fluid path including a sensor element for at least one of transmitting temperature and permitting conductivity sensing of fluid passing through the conduit, wherein the well is adapted for interconnection with a sensor.
2. A cassette according to statement 1, wherein the sensor element is a thermal well.
3. A cassette comprising a fluid path including a thermal well, said thermal well comprising:
   a hollow housing of a thermally conductive material, said housing having an outer surface and an inner surface, said inner surface of a predetermined shape so as to form a mating relationship with a sensing probe, whereby said mating thermally couples the inner surface with a sensing probe.
4. A method for determining temperature and conductivity of a subject media, said method comprising the steps of:
   providing a cassette containing at least one sensor element;
   thermally coupling a thermal well and a sensing probe such that temperature and conductivity can be determined;
   transferring thermal and conductivity signals through at least 3 leads from said sensing probe; and
   determining temperature and conductivity using said signals.
5. A cassette according to statement 1, wherein the sensor element is a thermal well.
6. Apparatus comprising a fluid path within a cassette including a well for at least one of transmitting temperature and permitting conductivity sensing of fluid passing through the conduit, wherein the well is adapted for interconnection with a sensor.
7. Apparatus according to statement 6, configured so that a portion of the well comes into contact with fluid in the fluid path.
8. Apparatus according to statement 7, wherein the well is coupled to the cassette using at least one of press fit connection, flexible tabs, adhesive, ultrasonic weld, and a retaining plate and fastener.
9. A fluid pumping cassette comprising at least one pump and a well for at least one of transmitting temperature and permitting conductivity sensing of fluid passing through the conduit, wherein the well is adapted for interconnection with a sensor.
10. A fluid pumping apparatus according to statement 9, wherein the at least one pump includes at least one pod pump.
11. A fluid pumping apparatus according to statement 9, wherein the at least one pump includes at least one pump chamber.
12. A sensing system for sensing subject media in a fluid path in a cassette comprising:
   a sensing probe; and
   a well, the well in communication with the sensing probe for at least one of thermal sensing and conductivity sensing.
13. A sensing system for sensing subject media comprising:
   a sensor manifold comprising at least one fluid port;
   sensor apparatus for sensing at least one characteristic of subject media in said fluid path.
14. A sensing system according to statement 13, wherein the sensor manifold further comprises a printed circuit board.
15. A sensing system according to statement 13, wherein the sensor manifold is comprised of a top plate and a mid plate.
16. A sensing system according to statement 13, wherein the sensor manifold is free of valves and pumping mechanisms.

## Claims

1. A sensor manifold (4100) for sensing liquid in a sensor apparatus, the sensor manifold comprising:
a base (4301) with a plurality of fluid channels (4220, 4221, 4223, 4224, 4225) in fluid communication with a plurality of tube connectors (4101, 4113);
a top plate (4302) with a plurality of ports, the top plate being sealingly engaged with the base to define the plurality of fluid channels, each of the plurality of ports configured to receive a sensing probe (4501) that extends into one of the plurality of fluid paths; and
a printed circuit board (PCB) (4502) electrically connected to each of the sensing probes;
wherein the top plate is positioned between the printed circuit board and the base, the PCB, top plate, and base are held together as a unit, and there are at least two sensing probes positioned in each fluid path for measuring conductivity of liquid in each fluid path.

2. The sensor manifold of claim 1 wherein at least one of the at least two sensing probes in each fluid path includes a temperature sensor (5808) thermally attached to a distal end of the sensing probe.

3. The sensor manifold of claim 2 wherein the temperature sensor is a thermistor.

4. The sensor manifold of claim 1, 2 or 3 wherein the PCB comprises an edge connector to electrically interconnect the sensor manifold to the sensor mechanism or controller of the medical apparatus.

5. The sensor manifold of any preceding claim wherein the PCB is attached to the top plate.

6. The sensor manifold of any preceding claim wherein both of the at least two sensing probes in each fluid path include a temperature sensor thermally attached to a distal end of the sensing probe.

7. The sensor manifold any preceding claim wherein the sensing probes are fabricated from one or more of the following materials: stainless steel, titanium, graphite and metal.

8. The sensor manifold of any preceding claim wherein the top plate includes a connector to secure and mechanically connect the sensor manifold to the medical apparatus.

9. The sensor manifold of any preceding claim wherein a PCB cover is attached to the PCB on the side opposite from the side of the PCB facing the top plate.

10. The sensor manifold of any preceding claim wherein the sensing probes each comprise a hollow housing of a thermally and electrically conductive material, said housing having an outer surface and an inner surface, said inner surface of a predetermined shape so as to form a mating relationship with a temperature sensor.

11. The sensor manifold of any preceding claim wherein the sensing probes are coupled to the top plate using at least one of press fit connection, flexible tabs, adhesive, ultrasonic weld, and a retaining plate and fastener.

12. The sensor manifold of any preceding claim wherein the sensing probes are sealed to the top plate with an o-ring, the o-ring having a cross-section with a cross-sectional shape that is round, square, or X-shaped.
